# EUROPEAN PATENT SPECIFICATION

(11) **EP 1 153 034 B1**
(45) Date of publication and mention of the grant of the patent: **17.10.2007**
(21) Application number: 00913536.9
(22) Date of filing: 18.02.2000
(51) Int. Cl.: C07K 5/062, A61K 38/05, A61P 19/00

(54) **GROWTH HORMONE SECRETAGOGUES**
SEKRETIONSFORDERMITTEL FUR WACHTUMSHORMON
SECRETAGOGUES D'HORMONE DE CROISSANCE

(30) Priority: 19.02.1999 US 120813 P
(43) Date of publication of application: 14.11.2001
(73) Proprietor: ELI LILLY AND COMPANY, Indianapolis, Indiana 46285 (US)
(72) Inventor: DODGE, Jeffrey, Alan, Indianapolis, IN 46236 (US); LUGAR, Charles, Willis, III, McCordsville, IN 46055 (US)
(74) Representative: Burnside, Ivan John
(86) International application number: PCT/US2000/004274
(87) International publication number: WO 2000/049037

(56) References cited:
- EP-A- 0 615 977
- EP-A- 0 662 481
- WO-A-98/16527
- WO-A-99/08697
- WO-A-99/08699
- US-A- 5 721 250

## Description

### Background of the Invention

Growth hormone is a secretory protein of the pituitary gland of animals having wide ranging developmental effects on the organism. Artificial manipulation of growth hormone levels has been demonstrated to have significant therapeutic utility. Human growth hormone supplementation has been shown to be an effective treatment for growth hormone deficiencies and their related disease states in humans. Apart from this application, studies have uncovered new and significant properties of growth hormone which lend further importance to the ability to control growth hormone levels. For example, recent clinical studies indicate that growth hormone supplementation may be useful in combating the maladies of aging in humans. Elevated growth hormone levels in animals have been shown to result in increased lean muscle mass. One application of this latter observation could result in higher production of leaner meat products or in the production of larger and/or stronger animals.

While growth hormone is naturally produced by the pituitary gland, the secretion of growth hormone into the bloodstream is controlled by a second protein, Growth Hormone Releasing Factor (GRF). This hormone is also commonly known in the art as somatocrinin, Growth Hormone Releasing Hormone (GHRH), and Growth Releasing Hormone (GRH).

There are two ways to approach the problem of increasing circulating levels of growth hormone: (1) increase the level of human growth hormone in the organism directly or (2) increase the organism's natural tendency to produce growth hormone. The latter strategy may be achieved via supplementation with GRF. GRF has been demonstrated to increase the circulatory levels of growth hormone *in vivo.* (Rivier, et al., Nature (London), 300:276 (1982). The effect of GRF, including structural analogs thereof, on growth hormone production has been widely studied. A primary obstacle to the use of GRF as a direct supplement is its short lifespan *in vivo.* L.A. Frohman, et al., Journal of Clinical Investigation, 78:906 (1986). More potent and/or longer lasting GRF molecules are therefore desirable for the development of effective human therapeutic or animal husbandry agents.

The structure of GRF has been modified in numerous ways resulting in longer lasting and/or more potent GRF analogs. It has been demonstrated that the first 29 amino acids from the N-terminus are sufficient to retain full GRF activity. Speiss, et al., Biochemistry, 21:6037 (1982). One strategy has been the incorporation of novel D-amino acid residues in various regions of the GRF molecule. V.A. Lance, et al., Biochemical and Biophysical Research Communications, 119:265. (1984); D.H. Coy, et al., Peptides, 8 (suppl. 1):49 (1986). Another strategy has modified the peptide backbone of GRF by the incorporation of peptide bond isosteres in the N-terminal region. D. Tourwe, Janssen. Chim. Acta, 3:3 (1985); S.J. Hocart, et al., Journal of Medicinal Chemistry, 33:1954-58 (1990). A series of very active analogs of GHRH is described in European Patent Publication 511,003, published October 28, 1992.

In addition to the actions of GHRH there are various ways known to release growth hormone. For example, chemicals such as arginine, L-3,4-dihydroxyphenylalanine (L-DOPA), glucagon, vasopressin, and insulin-induced hypoglycemia, as well as activities such as sleep and exercise, indirectly cause growth hormone to be released from the pituitary by acting in some fashion on the hypothalamus, perhaps either to decrease somatostatin secretion or to increase the secretion of GHRH.

In cases where increased levels of growth hormone are desired, the problem has generally been solved by providing exogenous growth hormone or by administering GHRH, or a related peptidyl compounds which stimulates growth hormone production or release. In either instance the peptidyl nature of the compound has necessitated that it be administered by injection.

Other compounds have been developed which stimulate the release of endogenous growth hormone, such as analogous peptidyl compounds related to GHRH. These peptides, while considerably smaller than growth hormones are still susceptible to metabolic instability.

Administration of the hexapeptide growth hormone releasing peptide-6 (GHRP-6) results in the secretion of growth hormone in many species, including humans.
This peptide is one of a series of synthetic peptides, the structures of which were based on the pentapeptide Met-enkephalin. It has been shown that GHRP binds specifically to the pituitary, although the binding does not involve the opioid, GHRH, or the somatostatin receptors.

In recent years significant efforts have been taken to develop nonpeptidyl analogs of this series of compounds. Such compounds, termed growth hormone secretagogues, should be orally bioavailable, induce the production or release of growth hormone, and act in concert, or synergistically with GHRH.

Representative growth hormone secretagogues are disclosed in United States Patent 3,239,345; United States Patent 4,036,979; United States Patent 4,411,890; United States Patent 5,206,235; United States Patent 5,248,841; United States Patent 5,310,737; United States Patent 5,310,017; European Patent Publication 144,230; European Patent Publication 513,974; Patent Cooperation Treaty Patent Publication WO 94/07486; Patent Cooperation Treaty Patent Publication WO 94/08583; Patent Cooperation Treaty Patent Publication WO 94/13696; 1996, United States Serial Number 08/700,206, filed August 20, 1996 (published as US 5773441) and Science, 260:1640-1643 (1993). EP 0662481 discloses synthetic dipeptide structures having a spiro-piperidine amino acid derived residue and are described to be useful as growth hormone secretagogues.

United States Patent 5,206,235, issued April 27, 1993, describes a series of benzolactam compounds typified by the following structure.

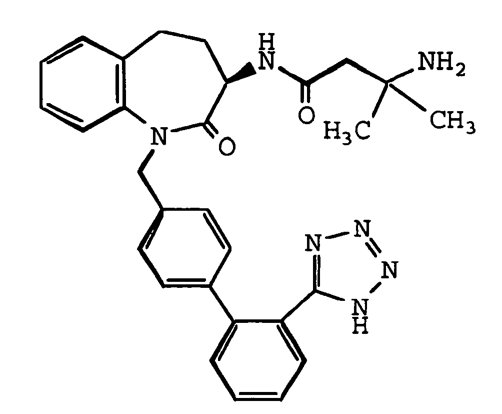

These compounds have demonstrated clinical activity in humans in raising the growth hormone secretory levels. B.J. Gertz, Journal of Clinical Endocrinology and Metabolism, 77:1393-1397 (1993).

Another group of growth hormone secretagogues is described in Patent Cooperation Treaty Patent Publication WO 94/13696, published June 23, 1994. These compounds are typified by the following two structures.

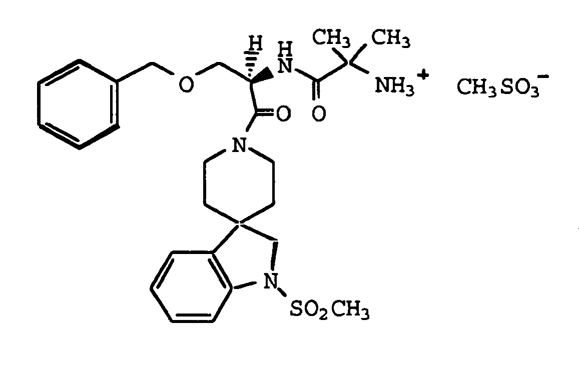

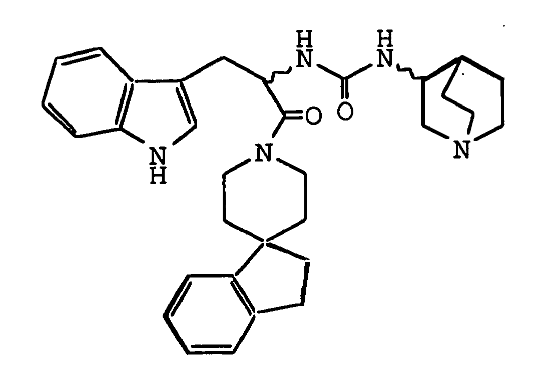

The present invention provides a series of compounds that have activity as growth hormone secretagogues. These compounds are non-peptidyl in nature and are, therefore, more metabolically stable than growth hormone, growth hormone releasing hormone, or analogs of either of these proteins. The compounds employed in the present invention are preferred for human pharmaceutical uses as well as veterinary uses, particularly in cattle, swine, sheep, poultry and fish.

### Summary of the Invention

The present invention relates to compounds of Formula I 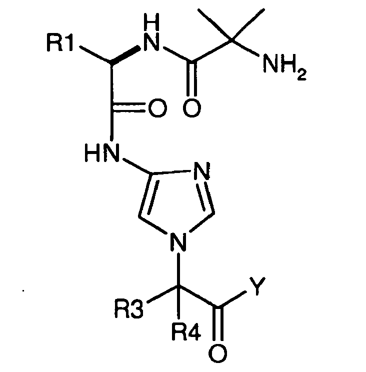 wherein:
R¹ is C₆H₅CH₂OCH₂- C₆H₅(CH₂)₃- or indol-3-ylmethyl;
Y is pyrrolidin-1-yl, 4-methyl piperidinyl or NR2R2;
R2 are each independently a C₁ to C₆ alkyl;
R3 is phenyl para-substituted by W;
W is F; and
R4 is CH₃,
or a pharmaceutically acceptable salt or solvate thereof.

The present invention further relates to pharmaceutical formulations containing compounds of Formula I, alone or in combination with other growth hormone secretagogue compounds, and/or in combination with suitable bone-antiresorptive agents, and the use of said compounds and/or formulations for the manufacture of a medicament for the treatment or prevention of congestive heart failure, osteoporosis and/or loss of muscle strength.

The present invention still further relates to processes for the preparation of compounds of Formula I.

### Detailed Description

In a preferred embodiment, compounds of the present invention are those compounds of Formula I wherein Y is 4-methylpiperidin-1-yl.

It is also preferred that the stereochemistry, at the two chiral centers, of the compounds of Formula I is (R,R).

The terms and abbreviations used in the instant examples have their normal meanings unless otherwise designated. For example "°C" refers to degrees Celsius; "N" refers to normal or normality; "mmol" refers to millimole or millimoles; "g" refers to gram or grams; "ml" means milliliter or milliliters; "M" refers to molar or molarity; "MS" refers to mass spectrometry; "FDMS" refers to field desorption mass spectrometry; "UV" refers to ultraviolet spectroscopy; "IR" refers to infrared spectroscopy; and "NMR" refers to nuclear magnetic resonance spectroscopy.

As used herein, the term "C₁-C₆ alkyl" refers to straight or branched, monovalent, saturated aliphatic chains of 1 to 6 carbon atoms and includes, but is not limited to, methyl, ethyl, propyl, isopropyl, butyl, isobutyl, t-butyl, pentyl, isopentyl, and hexyl. The term "C₁-C₆ alkyl" includes within its definition the term "C₁-C₄ alkyl".

"C₁-C₆ alkoxy" represents a straight or branched alkyl chain having from one to six carbon atoms attached to an oxygen atom. Typical C₁-C₆ alkoxy groups include methoxy, ethoxy, propoxy, isopropoxy, butoxy, t-butoxy, pentoxy and the like. The term "C₁-C₆ alkoxy" includes within its definition the term "C₁-C₄ alkoxy".

The term "carboxy-protecting group" as used herein refers to substituents of the carboxy group commonly employed to block or protect the carboxy functionality while reacting other functional groups on the compound. Examples of such protecting groups include methyl, ethyl, p-nitrobenzyl, p-methylbenzyl, p-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylene-dioxybenzyl, benzhydryl, 4,4'-dimethoxy-benzhydryl, 2,2',4,4'-tetramethoxybenzhydryl, t-butyl, t-amyl, trityl, 4-methoxytrityl, 4,4'-dimethoxytrityl, 4, 4', 4"-trimethoxytrityl, 2-phenylprop-2-yl, trimethylsilyl, t-butyldimethylsilyl, phenacyl, 2,2,2-trichloroethyl, 2-(di(*n*-butyl)methylsilyl)-ethyl, p-toluenesulfonylethyl, 4-nitrobenzylsulfonylethyl, allyl, cinnamyl, 1-(trimethylsilylmethyl)prop-1-en-3-yl, and the like.

A preferred carboxy-protecting group for the practice of the present invention is methyl or ethyl. Further examples of these groups may be found in E. Haslam, *supra,* at Chapter 5, and T.W. Greene, *et al., supra,* at Chapter 5.

The term "amino-protecting group" as used herein refers to substituents of the amino group commonly employed to block or protect the amino functionality while reacting other functional groups on the compound. Examples of such amino-protecting groups include formyl, trityl, phthalimido, trichloroacetyl, chloroacetyl, bromoacetyl, iodoacetyl, and urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, *n*-butoxycarbonyl, (NBoc) t-butoxycarbonyl, 1,1-diphenyleth-1-yloxycarbonyl, 1,1-diphenylprop-1-yloxycarbonyl, 2-phenylprop-2-yloxycarbonyl,2-(*p*-toluyl)-prop-2-yloxycarbonyl, cyclopentanyloxycarbonyl, 1-methylcyclopentanyloxycarbonyl, cyclohexanyloxycarbonyl, 1-methylcyclobexanyl" oxycarbonyl, 2-methylcyclo-hexanyloxycarbonyl, 2-(4-toluylsulfonyl)-ethoxycarbonyl, 2-(methylsulfonyl)ethoxycarbonyl, 2-(triphenyl-phosphino)-ethoxycarbonyl, fluorenylmethoxycarbonyl (FMOC), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)-benzyloxycarbonyl, isobornyloxycarbonyl, 1-piperidyloxycarbonyl, and the like; benzoylmethylsulfonyl group, 2-nitrophenylsulfenyl, diphenylphosphine oxide and like amino-protecting groups.

The amino-protecting group employed is usually not critical so long as the derivatized amino group is stable to the condition of subsequent reactions on other positions of the intermediate molecule, and may be selectively removed at the appropriate point without disrupting the remainder of the molecule including any other amino-protecting groups. A preferred amino-protecting group for the practice of the present invention is t-butoxycarbonyl (NBoc). Further examples of groups referred to by the above terms are described by E. Haslam, Protective Groups in Organic Chemistry, (J.G.W. McOmie, ed., 1973), at Chapter 2; and T.W. Greene and P.G.M. Wuts, Protective Groups in Organic Synthesis (1991), at Chapter 7.

The term "leaving group" (Q) refers to a group of atoms that is displaced from a carbon atom by the attack of a nucleophile in a nucleophilic substitution reaction. Suitable leaving groups include bromo, chloro, and iodo, benzenesulfonyloxy, methanesulfonyloxy, and toluene-sulfonyloxy. The term "leaving group" (Q) includes activating groups.

The term "activating group" as used herein refers a leaving group which, when taken with the carbonyl (-C=O) group to which it is attached, is more likely to take part in an acylation reaction than would be the case if the group were not present, as in the free acid. Such activating groups are well-known to those skilled in the art and may be, for example, succinimidoxy, phthalimidoxy, benzo-triazolyloxy, azido, or -O-CO-(C₄-C₇ alkyl).

The compounds used in the method of the present invention have two chiral centers. As a consequence of these chiral centers, the compounds of the present invention occur as diastereomers and mixtures of diastereomers. All asymmetric forms, individual isomers and combinations thereof, are within the scope of the present invention.

The terms "R" and "S" are used herein as commonly used in organic chemistry to denote specific configuration of a chiral center. The term "R" *(rectus)* refers to that configuration of a chiral center with a clockwise relationship of group priorities (highest to second lowest) when viewed along the bond toward the lowest priority group. The term "S" *(sinister)* refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in Nomenclature of Organic Compounds: Principles and Practice, (J.H. Fletcher, et al., eds., 1974) at pages 103-120.

In addition to the (R)-(S) system, the older D-L system is also used in this document to denote absolute configuration, especially with reference to amino acids. In this system, a Fischer projection formula is oriented so that the number 1 carbon of the main chain is at the top. The prefix "D" is used to represent the absolute configuration of the isomer in which the functional (determining) group is on the right side of the carbon atom at the chiral center and "L", that of the isomer in which it is on the left.

In order to preferentially prepare one optical isomer over its enantiomer, a number of routes are available. As an example, a mixture of enantiomers may be prepared, and then the two enantiomers may be separated. A commonly employed method for the resolution of the racemic mixture (or mixture of enantiomers) into the individual enantiomers is to first convert the enantiomers to diastereomers by way of forming a salt with an optically active acid or base. These diastereomers may then be separated using differential solubility, fractional crystallization, chromatography, or the like. Further details regarding resolution of enantiomeric mixtures may be found in J. Jacques, et al., Enantiomers, Racemates, and Resolutions, (1991).

During any of the following synthetic sequences it may be necessary or desirable to protect sensitive or reactive groups on any of the molecules concerned. This may be achieved by employing conventional protecting groups as described, *supra.*

The compounds of the present invention may be prepared by a number of routes, many of which are known to those of skill in the art. The particular order of steps to be employed in the synthesis of compounds of formula I is dependent upon the compound to be synthesized, the starting material employed, and the relative lability of the various substituted moieties. Examples of such synthetic routes may be found in Schemes I through IV provided below, as well as in the Examples.

One synthetic route to compounds of the present invention is provided in Scheme IA-IC below. The compounds of formula IV' and IV are commercially available, or may be prepared using techniques known in the art. A compound of Formula IV may be prepared from a compound of Formula IV' through an intermediate acid chloride prepared by standard methods using thionyl chloride or oxalyl chloride. Treatment of the resulting acid chloride with a bromine source, such as N-bromosuccinimide, followed by quenching of the acid chloride with ethanol, results in compounds of Formula IV. It is to be understood that the bromine group on the compound of Formula IV may in fact be any suitable leaving group (Q), as defined herein. This preparation is provided below in Scheme IA. 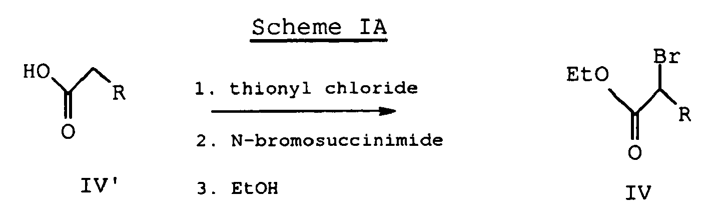 wherein R is representative of R3 as defined in a compound of Formula I above.

As shown in Scheme IB, the starting material further includes compounds of Formula V, which are commercially available, or may be routinely synthesized using techniques readily known in the art. Compounds of Formula IV may be coupled with a compound of formula V (4-nitroimidazole) by methods known in the art to generate a compound of Formula IIb'. Suitable agents to be employed in the coupling of these compounds include the treatment of a compound of Formula IV with an organic or inorganic base, followed by reaction with the bromo compound of Formula IV. Standard organic bases include trialkylamines, potassium hexamethyldisilazide, lithium hexamethyldisilazide, lithium diisopropylamide, potassium carbonate, and the like. Preferred for the practice of the present invention is sodium hydride or potassium carbonate in dimethylformamide.

A compound of Formula IIb' is then deprotected to provide a compound of Formula IIb, using lithium hydroxide, although other deprotecting reagents may be employed in this reaction. Such deprotecting agents include standard saponification reagents such as sodium hydroxide, potassium hydroxide, and lithium hydroxide.

Substantially pure (R) enantiomers of compounds of Formula IIb may also be synthesized by methods provided in U.S. 5,344,937 and 5,380,866.

A compound of Formula IIb is then converted to the corresponding amide under appropriate conditions with a compound of formula VI to generate a compound of Formula IIa. In general, amidation of primary or secondary amines of Formula VI may be accomplished by a number of methods known in the art in which activation of the acid to form a better leaving group. Suitable activating agents for this are also known in the art and include dicyclohexycarbodiimide (DCC), 1-(3-dimethylaminopropyl)-3-ethylcarbodiimide hydrochloride (EDC) with hydroxybenzotriazole (HOBT), oxalyl chloride, thionyl chloride, PyBOP^{®} (benzotriazol-1-yl-oxytripyrrolidine-phosphonium hexafluorophosphate), and the like. Preferred for the practice of the present invention is hydroxybenzotriazole (HOBT). The nitro group on the resulting compound of Formula IIa may then be reduced to an amino group using any suitable means, employing a suitable reducing agent. Preferred for the practice of the present invention is a catalytic reduction employing hydrogen and 5% palladium on carbon. A compound of Formula II is produced by this reduction reaction.

The preferred reaction temperature range employed in these reactions is between -40 and 150 °C, and the most preferred range is between 10 and 40 °C. These reactions may be conveniently carried out in situ, without isolation of the particular compound after its preparation.

Examples of these reactions are provided below in Scheme IB wherein R is representative of R3 as previously defined, and R₂R₁N is R₆ as previously defined.

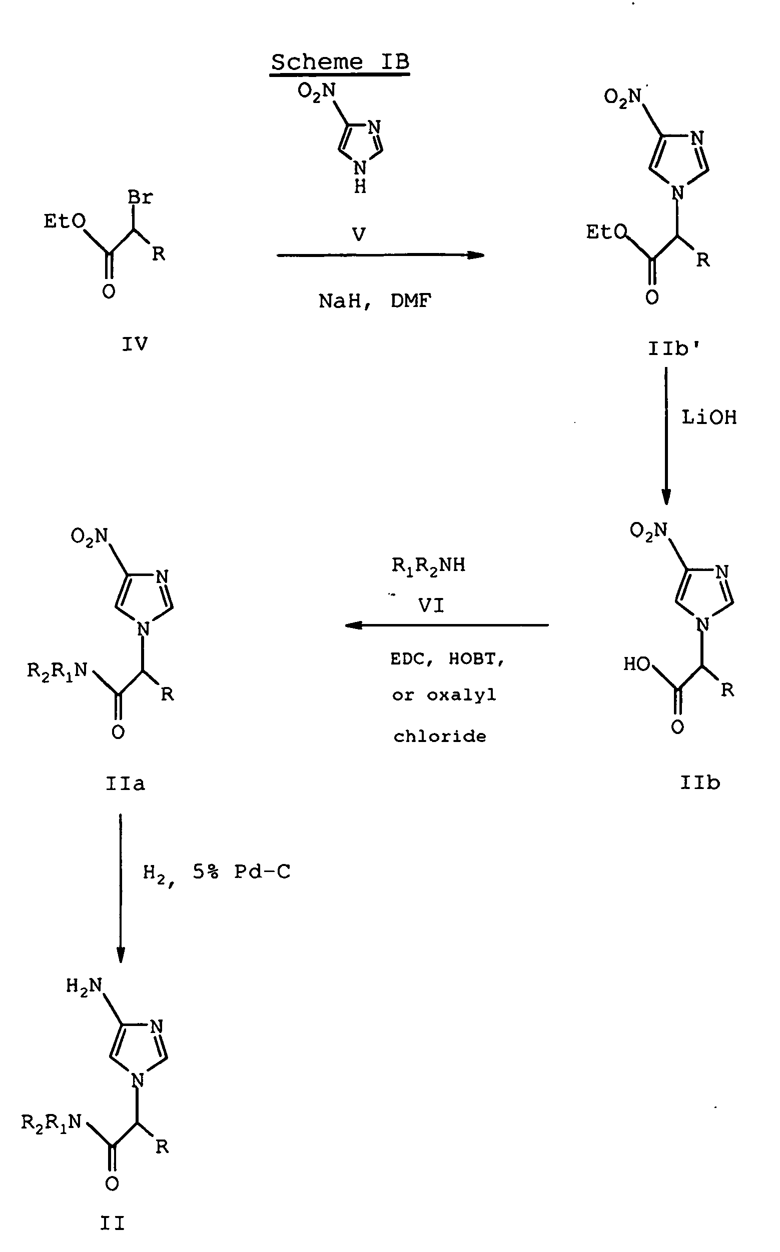

A second portion of the overall synthesis of compounds of Formula I is provided in Scheme IC below.

Representative starting material for this synthesis is a compound of Formula IIIb', which is a chemically-protected form of the amino acid O-serine. By chemically-protected it is meant that both the amino- and carboxy- functional groups have been suitably protected in order to facilitate further reactions with this molecule. Such protection reactions are known to those of skill in the art, and may be applied to other suitable starting materials. Intermediates of formula IIIb' are commercially available, or may be prepared by standard syntheses of amino acids. Such syntheses are well known to persons of ordinary skill in the art and are described, for example, in Chemistry and Biochemistry of Amino Acids, (G.C. Chapman ed., 1985). The protected amino group may be specifically deprotected using trifluoroacetic acid and methylene chloride to allow for further reactions with this amino functional group. This deprotection reaction results in a compound of Formula IIIb.

A compound of Formula IIIb may then be N-acylated with an amino-protected compound of formula X to produce a compound of Formula IIIa'. Suitable activating agents for this N-acylation reaction are known in the art and include DCC, HOBT, EDC, and oxalyl chloride. Preferred for the practice of the present invention is HOBT. Compounds of formula X are commercially available, or are readily prepared from suitable available starting materials. The protected carboxy group on the compound of Formula IIIa' is then selectively deprotected, typically using lithium hydroxide, to generate a compound of Formula III. Compounds of Formula III in which the starting material IIIb' is 2-Nboc-amino-pentanoic acid methyl ester may also be prepared by the route described in Scheme IC.

A compound of Formula III is then coupled with a compound prepared from the reduction of IIb' with hydrogen and a palladium catalyst employing a coupling reaction to generate a compound of Formula Ia. Again, typical reagents for this N-acylation are known in the art, and include DCC and HOBT, which is the preferred method of coupling employed in the practice of the present invention. A compound of Formula Ia is then selectively deprotected at the carboxy group, coupled at this site with a compound of Formula VI, and then further deprotected at the amino group to generate a compound of Formula Ia. Suitable agents for these deprotection and coupling reactions are discussed, *infra,* and are known in the art. Compounds of Formula Ia are encompassed by Formula I, and are pharmaceutically active.

The preferred reaction temperature range employed in these reactions is between -40 and 150 °C, and the most preferred range is between 10 and 40 °C. These reactions may be conveniently carried out *in situ,* without isolation of the particular compound after its preparation.

Alternatively, compounds of Formula IIa can be coupled with compounds of Formula III to provide intermediates which can be deprotected to give compounds of Formula Ia.

Representative reactions are provided below in Scheme IC, wherein R is R3 as previously defined, and R₂R₁N is R₆ as previously defined.

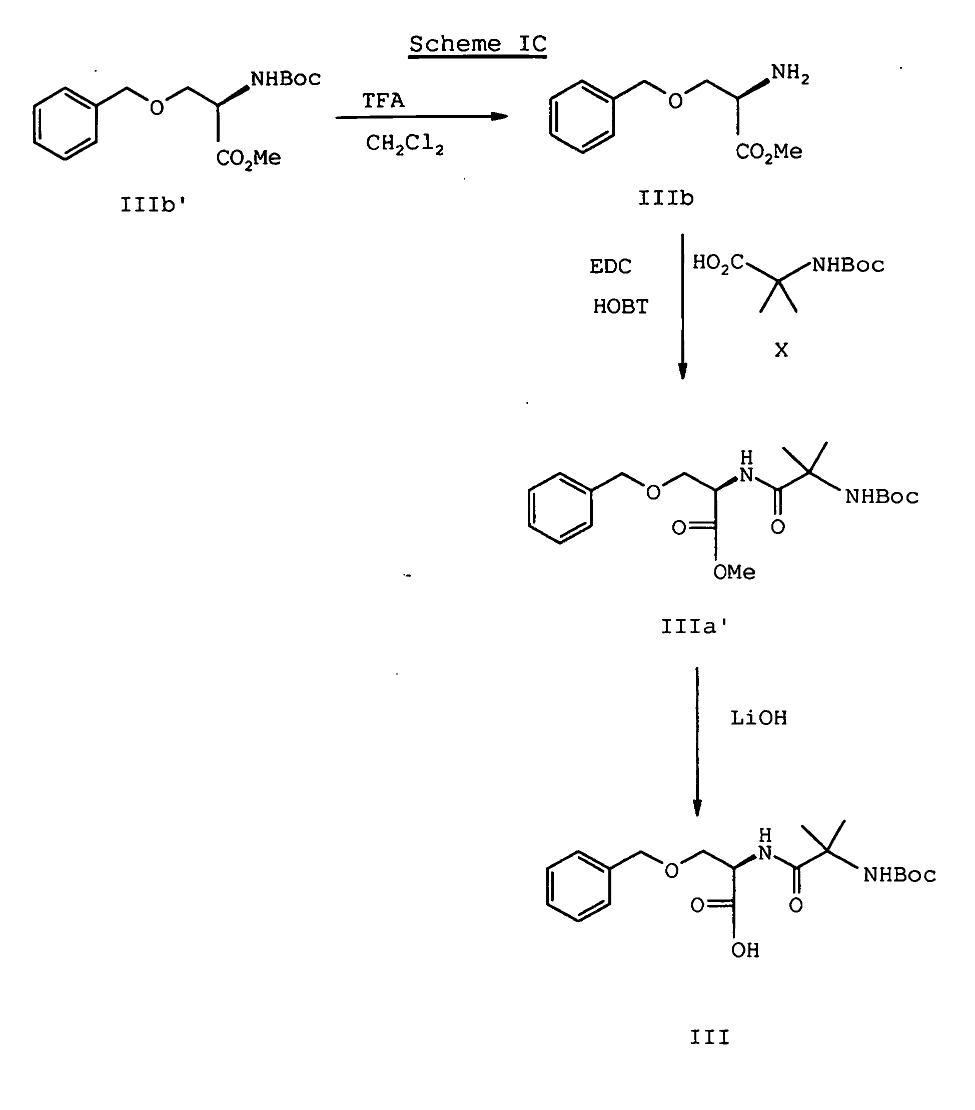

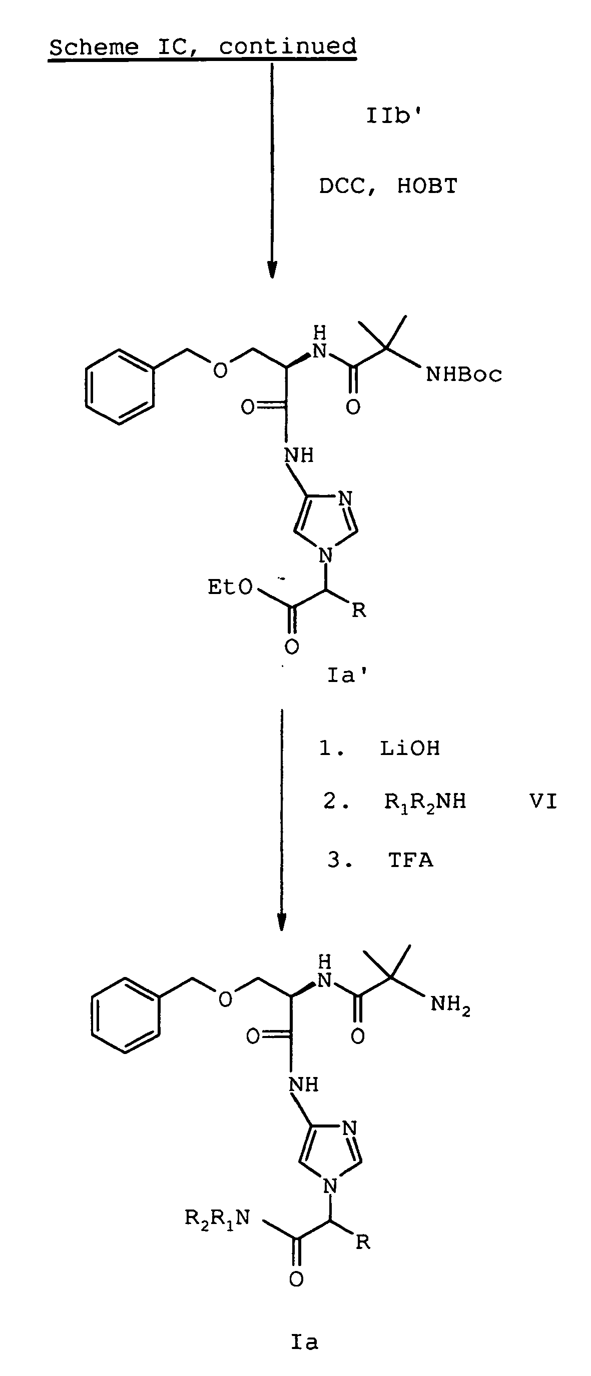

In addition to the synthetic scheme described hereinabove, an enantiospecific protocol for the preparation of the compounds of Formula I may be employed. Typically, a synthetic reaction design which maintains the chiral center present in the starting material in a desired orientation is chosen. The preferred reaction schemes are those that generally produce compounds in which greater than 95 percent of the product is the desired enantiomer. In Scheme II below, R-substituted phenyl is representative of the R₃ substituents as provided in compounds of Formula I above.

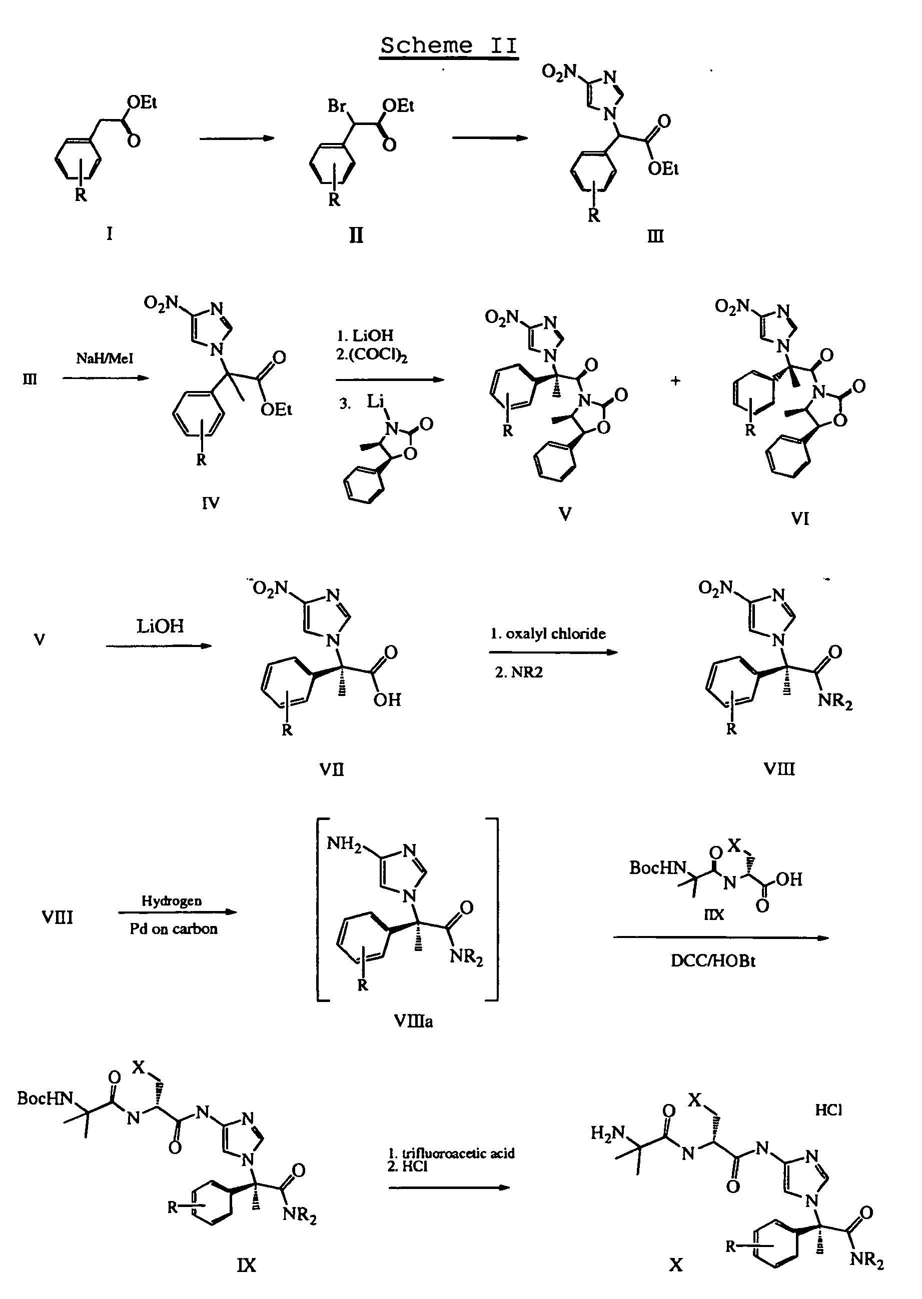

Within Scheme II, a compound of Formula IV may be prepared by the alkylation of a compound of formula III by standard methods using a base, such as sodium hydride, followed by treatment with an electrophile, such as methyl iodide. Preferred bases for this reaction include sodium-, lithium-, or potassium hexamethyldisilazide, lithium diisopropylamide, and sodium hydride. Preferred methylating agents include methyl halides or any methyl group with a suitable substituted leaving group such as tosylate, mesylate, and the like.

A compound of Formula V may be prepared by hydrolysis of a compound of Formula IV using standard saponfication conditions known in the art. Suitable reagents for this transformation include sodium hydroxide or lithium hydroxide. The resulting carboxylic acid may be converted into the acid chloride by standard methods using thionyl chloride or, preferably, oxalyl chloride. The acid chloride may then be reacted with the lithium salt of a chiral auxiliary, such as (4R, 5S)-(+)-4-methyl-5-phenyl-2-oxazolidinone, to provide compounds of formula V and VI, which are readily separable by silica gel chromatography.

A compound of Formula VII may be prepared by the removal of the chiral auxiliary under basic conditions, such as lithium hydroxide. Other reagents known in the art for removing oxazolidinone-type chiral auxiliaries may be used for this transformation. These include lithium hydroxide/hydroperoxide conditions, reduction/oxidation protocols, alkyl sulfur displacements, and transaminations.

A compound of Formula VIII may be prepared from a compound of Formula VII by standard methods known in the art. Formation of the acid chloride using oxalyl or thionyl chloride followed by reaction with a suitable substituted amine (NR₂) provide compounds of Formula VIII.

A compound of Formula IX may be prepared by the reduction of a compound of Formula VIII using hydrogen with palladium on carbon. Other methods known in the art which may be employed for the reduction of the nitro group include the use of tin(II)chloride, iron in an acidic solution, ferrous sulfate and aqueous alkali, activated alumina, and sodium sulfite. The resulting 4-amino imidazole compound of Formula VIIa is then reacted directly with the appropriate dipeptide acid (a compound of Formula IIX) under standard peptide coupling conditions involving formation of the active ester of the dipeptide followed by reaction with amine VIIa. Conditions suitable for amide formation include DCC, EDC, with HOBT. A compound of Formula IIX may be prepared from the methyl ester of unnatural D-amino acids such as D-benzyloxyserine, D-tryptophan, and D-2-amino-5-phenyl-pentanoic acid and the like which are known in the art. Standard coupling protocols involving formation of the active ester of the amino acid using DCC/HOBT followed by reaction with N-Boc-aminoisobutyric acid provide dipeptide acids of Formula IIX.

The Boc protecting group of a compound of Formula IX may be removed under standard acidic conditions such as hydrochloric acid in acetic acid or ethyl acetate, trifluoroacetic acid, tetramethyliodosilane, aluminum chloride, sulfuric acid in dioxane, and methanesulfonic acid.

An additional method of preparing diastereomeric compounds of Formula I involves the use of a chromatographic column which employs a chiral phase. An example of such a preparation may be found in Examples Part 6 as provided hereinbelow.
Two additional Schemes, which show general procedures of preparation, for providing chiral intermediates are provided hereinbelow as Schemes IIIA and IIIB. As described in Scheme IIIA, optically pure aryl glycine amino acids may be protected at the amino position by reaction with a suitable protecting group, such as Boc. Reaction of the Boc protected intermediate with a standard methylating agent, such as methyl iodide, may provide the corresponding phenolic methyl ether. The carboxamide may be prepared by coupling with an amine, such as dimethylamine, pyrrolidine, or 4-methyl piperidine, using standard coupling techniques. Preferred coupling agents for the invention are diethyl cyanophosphorane (DECP), triethylamine and the amine at 0°C. The Boc protecting group may be removed under standard acidic conditions, with trifluoroacetic acid being preferred. The desired 4-nitroimidazole compounds can be prepared by reaction of the free amine with 1, 4-dinitroimidazole to give optically pure compounds, as determined by chiral HPLC. Such chiral intermediates can be processed as described in Schemes I and II to provide diastereomerically pure products. For example, the chiral nitroimidazoles described in Scheme IIIA or IIIB may be reduced under standard conditions, such as hydrogenation with a palladium catalyst, to provide the corresponding chiral amino intermediate II. Such intermediates may be subsequently coupled with compounds of formula III of Scheme II as previously described to provide a chiral intermediate which can be deprotected to give diastereomerically pure compounds of formula Ia.

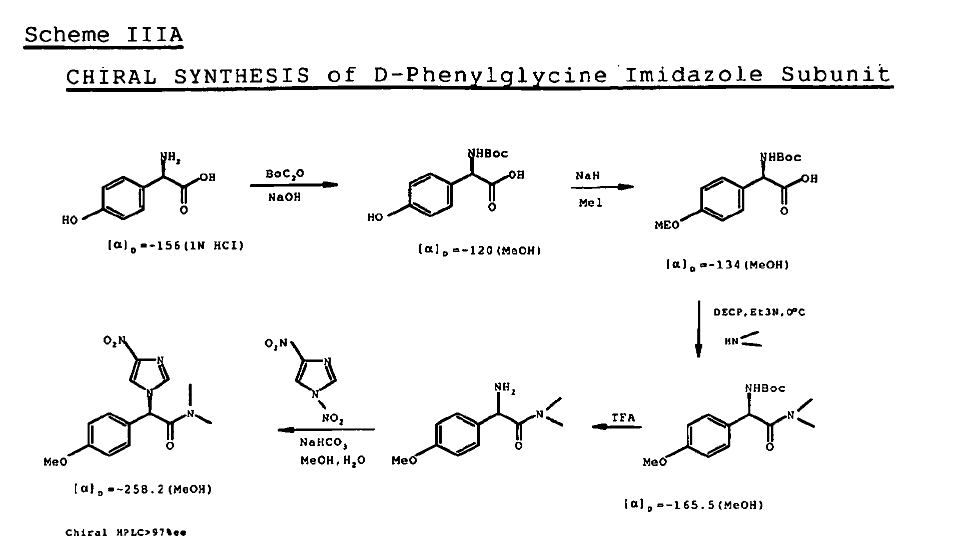

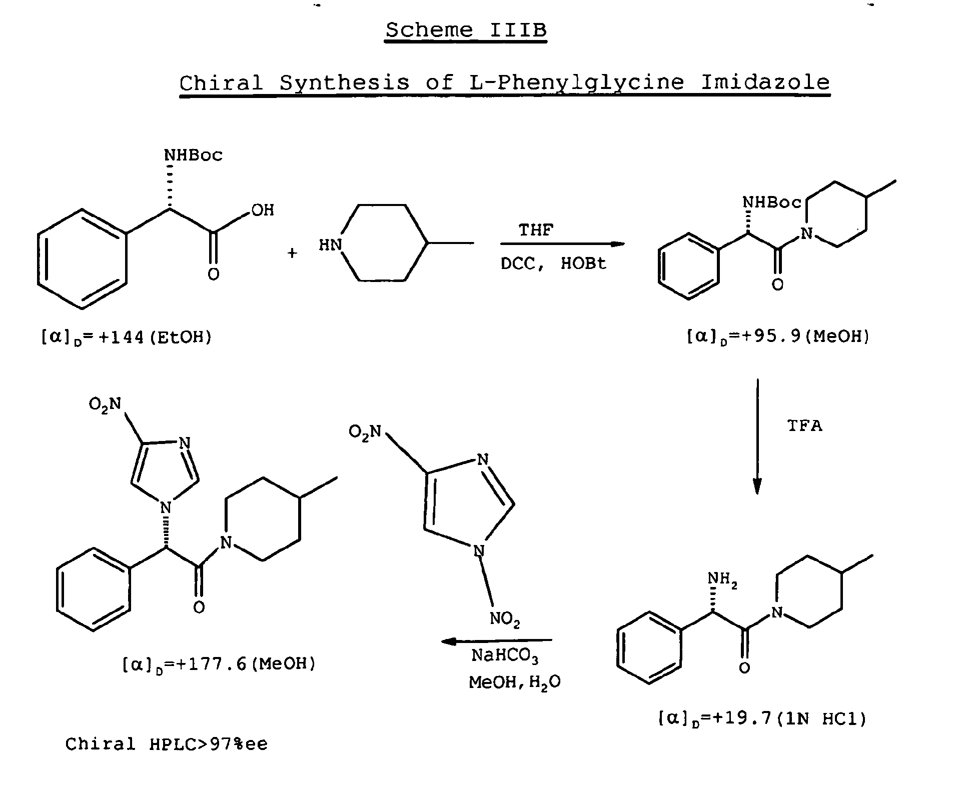

An additional approach and corresponding synthetic scheme for the general preparation of compounds of the instant invention is provided below in Scheme IV:

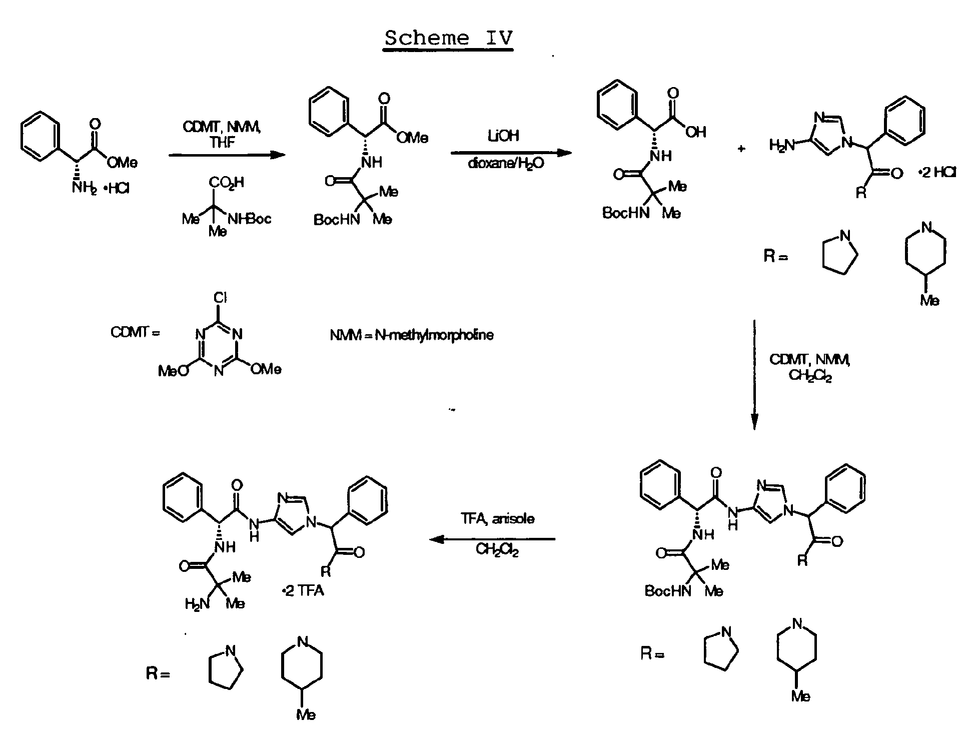

Compounds of Formula I may be conveniently screened for growth hormone secretagogue activity. A typical assay may employ pituitary cells established in culture, followed by a challenge with the various compounds of formula I, and the levels of growth hormone determined accordingly. Growth hormone levels may be calculated using various radioimmunoassay techniques known to those of skill in the art. Screening of compounds for growth hormone secretagogue activity may conveniently be scaled up for high throughput screening.

The invention further encompasses methods employing the pharmaceutically acceptable salts of the compounds defined by Formula I. Although generally neutral, a compound of this invention can possess a sufficiently acidic, a sufficiently basic, or both functional groups, and accordingly react with any of a number of inorganic bases, and inorganic and organic acids, to form a pharmaceutically acceptable salt.

The term "pharmaceutically acceptable salt" as used herein refers to salts of the compounds of Formula I which are substantially non-toxic to living organisms. Typical pharmaceutically acceptable salts include those salts prepared by reaction of the compounds of the present invention with a pharmaceutically acceptable mineral or organic acid or an inorganic base. Such salts are known as acid addition and base addition salts.

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic, methanesulfonic acid, oxalic acid, p-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, γ-hydroxybutyrate, glycolate, tartrate, methanesulfonate, propanesulfonate, naphthalene-1-sulfonate, naphthalene-2-sulfonate, mandelate, mesylate, and the like. Preferred pharmaceutically acceptable acid addition salts are those formed with mineral acids such as hydrochloric acid and hydrobromic acid, and those formed with organic acids such as maleic acid and methanesulfonic acid.

Salts of amine groups may also comprise quaternary ammonium salts in which the amino nitrogen carries a suitable organic group such as an alkyl, alkenyl, alkynyl, or aralkyl moiety.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counterion forming a part of any salt of this invention is not of a critical nature, so long as the salt as a whole is pharmacologically acceptable and as long as the counterion does not contribute undesired qualities to the salt as a whole.

This invention further encompasses methods employing pharmaceutically acceptable solvates of the compounds of Formula I. Many of the Formula I compounds can combine with solvents such as water, methanol, ethanol and acetonitrile to form pharmaceutically acceptable solvates such as the corresponding hydrate, methanolate, ethanolate and acetonitrilate.

As used herein, the term "effective amount" means an amount of compound of the instant invention which is capable of inhibiting, alleviating, ameliorating, treating, or preventing further symptoms in mammals, including humans, which may be due to decreased levels of endogenous growth hormone.

By "pharmaceutically acceptable formulation" it is meant that the carrier, diluent, excipients and salt must be compatible with the active ingredient (a compound of Formula I) of the formulation, and not be deleterious to the recipient thereof. Pharmaceutical formulations can be prepared by procedures known in the art. For example, the compounds of this invention can be formulated with common excipients, diluents, or carriers, and formed into tablets, capsules, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agar agar, calcium carbonate, and sodium bicarbonate; agents for retarding dissolution such as paraffin; resorption accelerators such as quaternary ammonium compounds; surface active agents such as cetyl alcohol, glycerol monostearate; adsorptive carriers such as kaolin and bentonite; and lubricants such as talc, calcium and magnesium stearate and solid polyethylene glycols. Final pharmaceutical forms may be: pills, tablets, powders, lozenges, syrups, aerosols, sachets, cachets, elixirs, suspensions, emulsions, ointments, suppositories, sterile injectable solutions, or sterile packaged powders, and the like, depending on the type of excipient used.

Additionally, the compounds of this invention are well suited to formulation as sustained release dosage forms. The formulations can also be so constituted that they release the active ingredient only or preferably in a particular part of the intestinal tract, possibly over a period of time. Such formulations would involve coatings, envelopes, or protective matrices which may be made from polymeric substances or waxes.

In addition, the growth hormone secretagogue compounds as disclosed herein may be administered to a patient in need of treatment in combination with other growth hormone secretagogues known in the art, and/or with a suitable bone anti-resorptive agent or agents for the prevention or treatment of osteoporosis and/or loss of muscle strength. Said suitable bone anti-resorptive agents include selective estrogen receptor modulators, bisphophonates, calcitonin, and hormone replacement therapeutic agents. Additionally, PTH may be administered in combination with said growth hormone secretagogues. Said combination therapy may be administered concomitantly or sequentially.

Suitable dosing ranges of compounds of Formula I include 0.01 µg/kg/day to 60 mg/kg/day.

The present invention relates to the use of a compound of Formula I for the manufacture of a medicament for the treatment or prevention of congestive heart failure.

The present invention additionally relates to pharmaceutical formulations containing a growth hormone secretagogue alone or in combination with additional therapeutic agents useful for the treatment or prevention of congestive heart failure.

The use of growth hormone secretagogue compounds, for the modulation of cardiac function and for the treatment or prevention of congestive heart failure, are described in copending U.S. Patent Application Serial No. 09/137,255, filed August 19, 1998 (published as US 632 93 42) titled "Treatment of Congestive Heart Failure With Growth Hormone Secretagogues".

The particular dosage of a compound required to treat, inhibit, or prevent the symptoms and/or disease of congestive heart failure in a mammal, including humans, according to this invention will depend upon the particular disease, symptoms, and severity. Dosage, routes of administration, and frequency of dosing is best decided by the attending physician. Generally, accepted and effective doses will be from 15mg to 1000mg, and more typically from 15mg to 80mg. Such dosages will be administered to a patient in need of treatment from one to three times each day or as often as needed for efficacy.

Representative pharmaceutical formulations containing compounds of formula I are provided below. The formulations which follow are given for purposes of illustration and are not intended to be limiting in any way. The total active ingredients in such formulations comprises from 0.1% to 99.9% by weight of the formulation. The term "active ingredient" means a compound of Formula I.

### Formulation 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

### Formulation 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

### Formulation 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

### Formulation 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose Polyvinylpyrrolidone | 35.0 mg |
| (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

### Formulation 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh U.S. sieve, and filled into hard gelatin capsules in 150 mg quantities.

### Formulation 6

Suppositories, each containing 25 mg of active ingredient are made as follows:

| Ingredient | Amount |
|---|---|
| Active Ingredient | 25 mg |
| Saturated fatty acid glycerides | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

### Formulation 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

### Formulation 8

Capsules, each containing 15 mg of medicament, are made as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 425 mg quantities.

### Formulation 9

An intravenous formulation may be prepared as follows:

| Ingredient | Quantity |
|---|---|
| Active Ingredient | 250.0 mg |
| Isotonic saline | 1000 ml |

### Formulation 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

### Formulation 11

Sublingual or buccal tablets, each containing 10 mg of active ingredient, may be prepared as follows:

| Ingredient | Quantity Per Tablet |
|---|---|
| Active Ingredient | 10.0 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another formulation employed in the methods of the present invention employs transdermal delivery devices or patches. Such transdermal patches may be used to provide continuous or discontinuous infusion of the compounds of the present invention in controlled amounts. The construction and use of transdermal patches for the delivery of pharmaceutical agents is well known in the art. See, for example, U.S. Patent 5,023,252.

Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5, 011, 472.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

The following Examples and Preparations are for reference purposes and are illustrative of the processes employed in the synthesis of the compounds of the present invention. As would be understood by persons skilled in the art, other synthetic schemes may be employed to prepare the compounds of the instant invention.

### Exemplification

### Example 1

### Preparation of Chemical Intermediates

### Example 1A

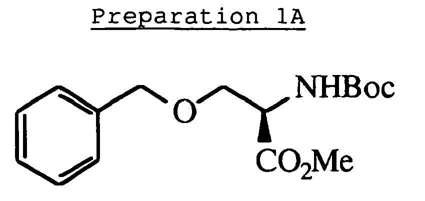

To a solution of tert-butyloxycarbonyl-O-benzyl (boc-OBz)-D-Ser-OH (25.0 g, 84.7 mmol), while stirring in anhydrous N,N-dimethylformamide (500 mL) at room temperature, was added sodium bicarbonate (14.2 g, 169 mmol) followed by methyl iodide (26.4 mL, 424 mmol). After 18 hours, the reaction mixture was concentrated to approximately 100 mL. Ethyl acetate was added and the mixture washed with aqueous sodium bicarbonate and brine. The organic extract was dried and concentrated to give the above-identified product (25 g, 96%) as a light yellow oil: ¹H NMR (300 MHz, CDCl₃) d 1.45 (s, 9H), 3.70 (m, 1H), 3.75 (s, 3H), 3.85 (m, 1H), 4.50 (m, 3H), 7.30 (m, 5H); MS (FD) m/e 310; Anal. calc'd for C₁₆H₂₃NO₅ : C, 62.12 ; H, 7.49; N, 4.53. Found: C, 62.31; H, 7.49; N, 4.43.

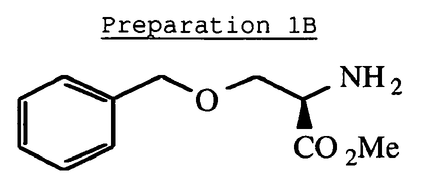

To a solution of a compound of Preparation 1A (5.0 g, 16 mmol), stirring in dichloromethane (25 mL) and anisole (1 mL) at 0 °C was added trifluoroacetic acid. After 4 hours at room temperature, saturated sodium bicarbonate solution was added and the mixture extracted with ethyl acetate. The combined organic extracts were washed with brine, dried over sodium sulfate, and concentrated. The above-identified crude product was used in the next step without further purification.

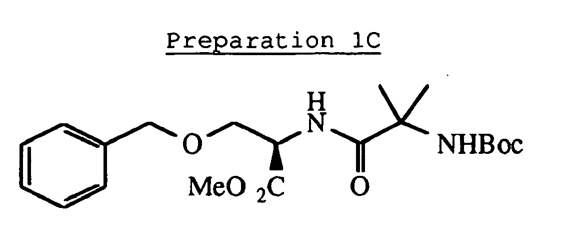

To a solution of a compound of Preparation 1B (65.4 mmol), boc-α-aminoisobutyric acid (13.2 g, 65.4 mmol), 1-hydroxybenzotriazole (8.8 g, 65.4 mmol), and N,N-diisopropylethylamine (22.8 mL, 130.7 mmol) stirring in dichloromethane (500 mL) at 0 °C was added 1-(3-dimethylaminopropyl)-3-ethylcarbodiimide (12.3 g, 71.9 mmol). After 18 hours, ethyl acetate and saturated ammonium chloride were added and the mixture extracted with ammonium chloride, sodium bicarbonate, and brine. The organic extracts were dried over sodium sulfate and concentrated. Purification by silica gel chromatography (25% ethyl acetate/hexanes) yielded the above-identified product (21.6 g, 83%) as a white solid: ¹H NMR (300 MHz, CDCI₃) d 1.39 (s, 9H), 1.48 (s, 6H), 3.62 (dd, J = 3.4, 9.1 Hz, 1H), 3.70 (s, 3H), 3.85 (dd, J = 3.4, 9.1 Hz, 1H), 4.48 (dd, J = 12.5, 22.7 Hz, 2H), 4.75 (m, 1H), 4.92 (s, 1H), 7.11 (d, J = 8.6 Hz, 1H), 7.35 (m, 5H); MS (FD) m/e 395; Anal. calc'd for C₂₀H₃₀N₂O₆: C, 60.90; H, 7.67; N, 7.10. Found: C, 61.02; H, 7.78; N, 7.10.

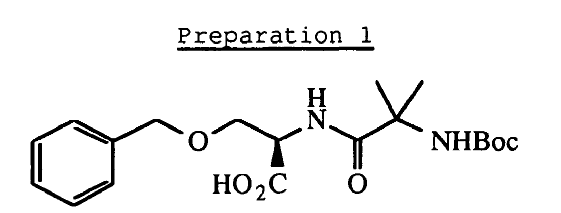

To a solution of a compound of Preparation 1C (5.30 g, 13.4), stirring in dioxane (100 mL)/water (50 mL) at room temperature was added lithium hydroxide (2.80 g, 67.3 mmol). After 18 hours, water was added and the solution concentrated. The resulting mixture was extracted with diethyl ether. Sodium chloride was added to the aqueous layer and the pH adjusted to 3.5 with 1 N HCl. The resulting mixture was extracted with ethyl acetate and the combined organic extracts dried over sodium sulfate then concentrated to yield the above-identified product (4.40 g, 86%) as a white foam: ¹H NMR (300 MHz, CDCl₃) d 1.39 (s, 9H), 1.45 (s, 3H), 1.47 (s, 3H), 3.68 (m, 1H), 3.95 (m, 1H), 4.54 (s, 2H), 4.70 (m, 1H), 5.51 (bs, 1H), 7.18 (d, J = 9.1 Hz, 1H), 7.25 (m, 5H), 9.90 (bs, 1H) ; MS (FD) m/e 381; Anal. calc'd for C₁₉H₂₈N₂O₆: C, 59.99; H, 7.42; N, 7.36. Found: C, 59.74; H, 7.26; N, 7.30.

### Example 1B

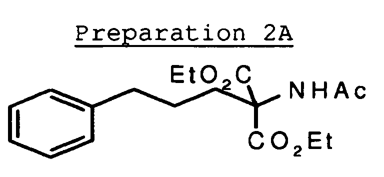

A solution of sodium ethoxide was generated by the addition of sodium metal (52.89 grams, 2.3007 mol), over 3 hours, to ethanol (1500 mL). To the sodium ethoxide solution, at ambient temperature, was added a solution of diethylacetamidomalonate (499.75 grams, 2.3007 mol) dissolved in ethanol (225 mL). The reaction mixture was stirred for 1.5 hours at ambient temperature. 1-bromo-3-phenylpropane (458.07 grams, 2.3007 mol) was then added over 15 minutes and the reaction mixture was refluxed until complete as determined by HPLC (16 hours). The reaction mixture was concentrated to dryness and the residue partitioned between ethyl acetate (1 x 1500 mL and 2 x 500 mL) and water (1500 mL). The ethyl acetate layers were combined, washed with saturated sodium chloride solution (4 x 500 mL), dried using sodium sulfate, and concentrated to give 752.1 grams (98%) of the above-identified product as a light yellow solid: A 1.0 gram sample was recrystallized from hexane:ethyl acetate (19:1, v:v) to give a mp 84-86°C. ¹H nmr (CDCl₃): δ 1.18-1.23 (t, 6H), 1.37-1.50 (m, 2H), 2.02 (s, 3H), 2.34-2.41 (m, 2H), 2.58-2.62 (t, 2H), 4.16-4.24 (q, 4H), 6.76 (s, broad, 1H), 7.11-7.28 (m, 5H). ¹³C nmr (CDCI₃): δ 13.95, 23.03, 25.67, 31.85, 35.45, 62.46, 66.49, 125.40, 125.90, 128.27, 128.35, 141.77, 168.11, 168.94. MS (FIA) m/z 336.3 ([M+H]⁺). IR (KBr, cm⁻¹) 1645.98 (amide), 1744.76 (C=O). Anal. Calc'd. for C₁₈H₂₅NO₅: C, 64.46; H, 7.51; N, 4.17. Found: C, 64.60; H, 7.37; N, 4.39.

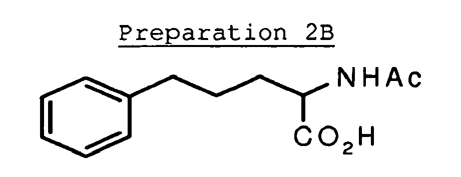

A slurry consisting of the product of Preparation 2A (249.15 grams, 0.7428 mol) and 2.5 N sodium hydroxide solution was then heated at 100 °C for three hours. The reaction mixture was cooled to 30°C and the pH adjusted to 5.0 using concentrated hydrochloric acid. The solution was then heated to 100 °C and the pH was held at 5.0 using concentrated hydrochloric acid as needed until the reaction was complete as determined by HPLC. The solution was filtered while hot through diatomaceous earth. The filtrate was cooled to 5-10 °C and the pH adjusted to 1.0 using concentrated hydrochloric acid. The resulting slurry was stirred for 1 hour at 5 °C, filtered, and dried in vacuum at 50 °C to give 160.34 grams (92%) of above-identified product, (DL)-N-acetyl-2-amino-5-phenylpentanoic acid, as a white powder, mp 145-148 °C. ¹H nmr (DMSO-d₆) : δ 1.60-1.71 (m, 4H), 1.86 (s, 3H), 2.56-2.59 (m, 2H), 4.19-4.23 (m, 1H), 7.16-7.30 (m, 5H), 8.14 (d, 1H). ¹³C nmr (DMSO-d₆) : δ 23.17, 28.25, 31.55, 35.51, 52.55, 126.60, 129.14, 142.64, 170.25, 174.65. MS (FIA) m/z 236.2 (M⁺). IR (KBr, cm⁻¹) 1609.17 (amide), 1741.12 (C=O). Anal. Calc'd. for C₁₃H₁₇NO₃: C, 66.36; H, 7.28; N, 5.95. Found: C, 66.41; H, 7.15; N, 5.96.

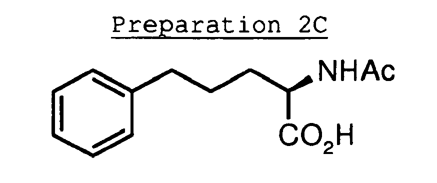

A solution consisting of (DL)-N-acetyl-2-amino-5-phenylpentanoic acid (Preparation 2B) (-438.0 grams, 1.862 mol), cobalt chloride (1.10 grams), 2N potassium hydroxide solution (931 mL, 1.862 mol), and water (8000 mL) was adjusted to a pH of 8.0 by the addition of 2N potassium hydroxide solution. To the reaction mixture was added Acylase I (aspergillus melleus, 39.42 grams) which was then vigorously stirred for 24 hours at 40 °C while maintaining a pH of 8.0 by addition of 2N potassium hydroxide. The resulting slurry was filtered. The filtrate was adjusted to a pH of 2.0 giving a thick slurry. The product was isolated by filtration, washed with hexane (2000 mL) and dried in vacuum at 50 °C to give 188.52 grams (43%) of the above-identified product, (D)-N-acetyl-2-amino-5-phenylpentanoic acid: ¹H nmr (DMSO-d₆): δ 1.59-1.74 (m, 4H), 1.86 (s, 3H), 2.57-2.60 (m, 2H), 4.22-4.26 (m, 1H), 7.16-7.30 (m, 5H), 8.02 (d, 1H), 12.39 (s, broad, 1H). ¹³C nmr (DMSO-d₆): δ 23.18, 28.13, 31.66, 35.54, 52.58, 126.56, 129.10, 142.67, 170.12, 174.48. MS (FIA) m/z 236.1 (M⁺). IR (KBr, cm⁻¹) 1625.08 (amide), 1700.24 (C=O). Anal. Calc'd. for C₁₃H₁₇NO₃: C, 66.36; H, 7.28; N, 5.95. Found: C, 66.49 ; H, 7.00; N, 6.03.

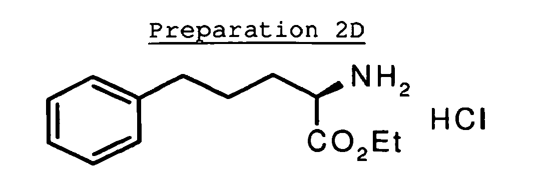

A solution consisting of (D)-N-acetyl-2-amino-5-phenylpentanoic acid (Preparation 2C)(188.8 grams, 0.8024 mol), ethanol (535 mL), and concentrated hydrochloric acid (268 mL, 3.21 mol) was warmed to 85 °C. The reaction was determined to be incomplete by HPLC at 14.5 hours and additional concentrated hydrochloric acid (50 mL) was then added. This reaction was determined to be complete by HPLC after 22.5 hours. Subsequently, water was azeotropically distilled from the reaction by continuous addition and distillation of 8000 mL of ethanol. The ethanol was azeotropically distilled from the reaction by the continuous addition and distillation of ethyl acetate (2000 mL). Upon cooling the solution to 0 °C the product crystallized. The solution containing the product was stirred for 1 hour at 0°C, filtered, and the cake dried in vacuum at 40 °C to give 199.0 grams (96%) of the above-identified product, 2-amino-5-phenylpentanoic acid, ethyl ester hydrochloride: (mp 117-121 °C. ¹H nmr (DMSO-d₆) : δ 1.15-1.21 (t, 3H), 1.50-1.89 (m, 4H), 2.48-2.67 (m, 2H), 3.92-3.98 (t, 1H), 4.08-4.25 (m, 2H), 7.12-7.29 (m, 5H), 8.76 (s, broad, 3H). ¹³C nmr (DMSO-d₆) : δ 13.90, 25.97, 29.52, 34.41, 51.71, 61.56, 124.91, 125.81, 128.24, 141.27, 169.35. MS (FIA) m/z 222.3 (M⁺) . IR (KBr, cm⁻¹) 1741.14 (C=0). [α]²⁰_{D} = -11.17(c = 30.62 mg / 3mL, MeOH). Anal. Calc' d. for C₁₃H₂₀NO₂Cl: C, 60.58; H, 7.82; N, 5.43. Found: C, 60.45; H, 7.67; N, 5.55.

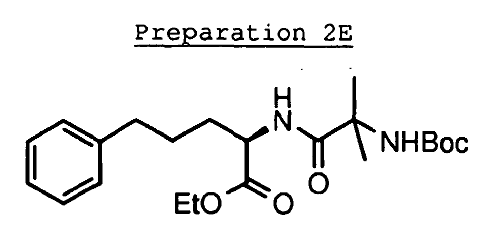

A slurry consisting of N-t-BOC-α-aminoisobutyric acid (90.64 grams, 0.446 mol), 2-chloro-4,6-dimethoxy-1,3,5-triazine (75.90 grams, 0.425 mol), N-methyl morpholine (88.13 grams, 0.871 mol), and diethyl ether (1000 mL) was stirred at ambient temperature until complete as determined by HPLC (3 hours). The D-2-amino-5-phenylpentanoic acid, ethyl ester hydrochloride (Preparation 2D), (109.55 grams, 0.425 mol) was added and the reaction mixture stirred for 16 hours at ambient temperature. The reaction mixture was partitioned between 10% citric acid solution (1000 mL) and ethyl acetate (3 x 500 mL). The organic phase was washed with 10% citric acid solution (3 x 500 mL), saturated sodium bicarbonate solution (3 x 500 mL), water (1 x 500 mL), dried using sodium sulfate, and concentrated to dryness. The residue was recrystallized from hexane (3000 mL) to give 155.11 grams of the above-identified product: mp 97-99 °C. ¹H nmr (CDCl₃) : δ 1.25-1.28 (t, 3H), 1.43 (s, 9H), 1.48 (s, 3H), 1.50 (s, 3H), 1.70-1.73 (m, 3H), 1.87-1.93 (m, 1H), 2.62-2.67 (m, 2H), 4.16-4.21 (m, 2H), 4.57-4.62 (m, 1H), 4.95 (s, 1H), 6.96 (s, broad, 1H), 7.16-7.19 (m, 3H), 7.26-7.33 (m, 2H). ¹³C nmr (CDCl₃) : δ 14.53, 26.32, 27.17, 28.67, 32.47, 35.73, 52.54, 57.17, 61.62, 126.21, 128.69, 128.79, 142.12, 154.99, 172.81, 174.69. MS (FIA) m/z 407.5 ([M+H]⁺). IR (KBr, cm⁻¹) 1652.75, 1685.52 (amides), 1741.73 (C=O) . [α]²⁰_{D} = 7.83 (c = 10.22 mg / 1mL, MeOH). UV (0.1% trifluoroacetic acid in water : acetonitrile) λₘₐₓ 215.6 nm. Anal. Calc'd. for C₂₂H₃₄N₂O₅: C, 65.00; H, 8.43; N, 6.89. Found: C, 65.23; H, 8.34; N, 6.94.

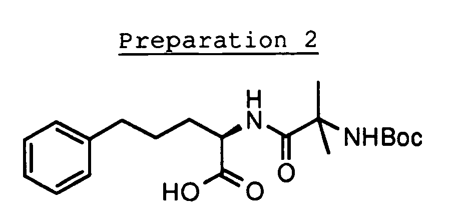

A solution consisting of the product of Preparation 2E (152.53 grams, 0.3752 mol) and tetrahydrofuran (884 mL) was cooled to 5 °C. A solution consisting of lithium hydroxide (26.96 grams, 1.126 mol) and water (1419 mL) was added to the reaction dropwise over 10 minutes while maintaining a temperature of 5-10 °C. Ethanol (183 mL) was added and the reaction stirred at 5-10 °C until complete as determined by HPLC (2 hours). The pH of the reaction mixture was then adjusted to 2.0 using 6 N hydrochloric acid solution while maintaining 5-10 °C. The product was extracted from solution with ethyl acetate (3 x 500 mL). The ethyl acetate extracts were combined, dried using sodium sulfate, and concentrated to dryness to give 141.51 grams (100%) of the above-identified product: ¹H nmr (DMSO-d₆): δ 1.32-1.37 (m, 15H), 1.57-1.75 (m, 4H), 2.51-2.58 (m, 2H), 4.23-4.27 (m, 1H), 6.85 (s, broad, 1H), 7.15-7.28 (m, 5H), 7.42 (d, 1H), 12.5 (s, broad, 1H). ¹³C nmr (DMSO-d₆) : δ 26.31, 27.85, 29.00, 31.86, 35.60, 52.53, 56.60, 78.95, 126.52, 129.05, 129.10, 142.69, 155.06, 174.40, 175.17. MS (FIA) m/z 379.5 ([M+H]⁺). IR (KBr, cm⁻¹) 1641.98, 1692.22 (amides), 1719.72 (C=O). [a]²⁰_{D} = -5.73 (c = 10.48 mg / 1mL, MeOH). Anal. Calc'd. for C₂₀H₃₀N₂O₅: C, 63.47; H, 7.99; N, 7.40. Found: C, 63.25; H, 7.84; N, 7.46.

### Example 1D

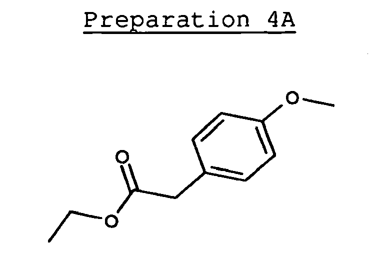

To a solution of 4-methoxyphenylacetic acid (98 g, 590 mmol), in absolute ethanol (300 mL), was added of p-toluenesulfonic acid (20 g, 105 mmol). The reaction mixture was heated to reflux and maintained at that temperature for 5 hours then cooled to room temperature and concentrated to dryness. The resulting oil was purified by flash chromatography (silica gel, 20% ethyl acetate/hexanes) to give 102 g (89%) of the above-identified product as a colorless oil: ¹H-NMR (d, DMSO) 1.17 (t, J = 8.7 Hz, 3H), 3.56 (s, 2H), 3.73 (s, 3H), 4.05 (q, J = 7.2 Hz, 2H), 6.87 (d, J = 8.7 Hz, 2H), 7.17 (d, 8.7 Hz, 2H); MS (ion spray) 195.3 (M+1) ; Anal. Calc'd for C₁₁H₁₄O₃: C, 68.02; H, 7.27. Found: C, 67.95, 7.17.

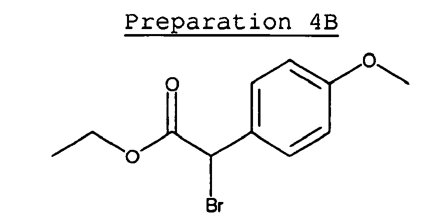

To a solution of the product of Preparation 4A (40 g, 200 mmol) in carbon tetrachloride (500 mL) was added N-bromosuccinimide (37 g, 206 mmol) and hydrobromic acid (4 drops of 48% aqueous solution). The resulting mixture was heated to reflux and maintained at that temperature for 5 hours then cooled to room temperature, filtered, and concentrated. The resulting oil was purified by flash chromatography (silica gel, chloroform) to give 51.1 g (94%) of the above-identified product as a colorless oil: ¹H-NMR (d, DMSO) 1.19 (t, J = 8.4 Hz, 3H), 3.77 (s, 3H), 4.18 (m, 2H), 5.88 (s, 1H), 6.95 (d, J = 8.4 Hz, 2H), 7.50 (d, J = 8.4 Hz, 2H); MS (FD) 272, 274 (M+) ; Anal. Calc'd for C₁₁H₁₃BrO₃: C, 48.37; H, 4.80. Found: C, 48.52, 4.77.

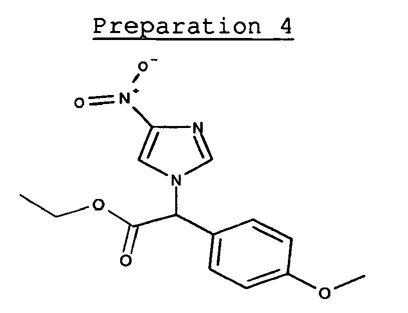

To a solution of the product of Preparation 4B (49.5 g, 181 mmol), stirring in dimethylformamide (500 mL) at room temperature, was added 4-nitroimidazole (20.5 g, 181 mmol) and potassium carbonate (75 g, 543 mmol). After 16 hours, the reaction mixture was filtered and concentrated. The resulting oil was partitioned between ethyl acetate and water and extracted with ethyl acetate. The combined organic extracts were washed with brine, dried over sodium sulfate, filtered and concentrated. The resulting oil was purified by flash chromatography (silica gel, 30-70% ethyl acetates/hexanes gradient) to yield 33.6 g (61%) of the above-identified product as an orange oil that solidifies upon standing: ¹H-NMR (d, DMSO) 1.17 (t, J = 7.2 Hz, 3H), 3.78 (s, 3H), 4.25 (q, J = 7.2 Hz, 2H), 6.57 (s, 1H), 7.02 (d, J = 8.7 Hz, 2H), 7.46 (d, J = 8.7 Hz, 2H), 7.92 (s, 1H), 8.38 (s, 1H); MS (ion spray) 306 (M+1) ; Anal. Calc'd for C₁₄H₁₅N₃O₅: C, 55.08; H, 4.95; N, 13.76. Found: C, 54.93; H, 4.89; N, 13.82.

### Example 1E

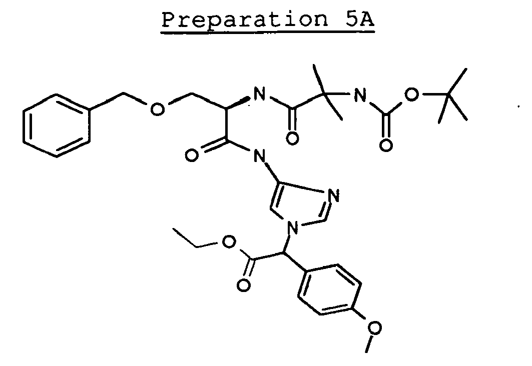

To a slurry of 10% palladium on carbon (6.0 g) was added a slurry of.the product of Preparation 4 (8.4 g, 27.5 mmol) in tetrahydrofuran (30 mL). The reaction mixture was placed under a hydrogen atmosphere (40 mm Hg) using a Parr apparatus, until the reduction was complete. The reaction mixture was then filtered through celite. To the resulting solution, stirring at room temperature, was added the product of Preparation 1 (10.5 g, 27.5 mmol), 1-hydroxybenzotriazole (4.1 g, 30.3 mmol) and 1-(3-dimethylaminopropyl)-3-ethylcarbodiimide (6.3 g, 30.3 mmol). After 16 hours, the reaction mixture was concentrated and the resulting oil was slurried in ethyl acetate and filtered. The solution was diluted with water and then extracted with ethyl acetate. The combined organic extracts were washed with brine, dried over sodium sulfate, filtered and concentrated. The resultant crude material was purified by flash chromatography (silica gel, 3% methanol/chloroform) to give 14.4 g (83%) of the above-identified product as a tan foam: ¹H-NMR (d, DMSO) 1.78 (t, J = 7.2 Hz, 3H), 1.27-1.32 (m, 15H), 3.60 (m, 1H), 3.67 (m, 1H), 3.76 (s, 3H), 4.20 (d, J = 7.2 Hz, 2H), 4.44 (d, J = 3.0 Hz, 2H), 4.57 (m, 1H), 6.35 (s, 1H), 6.97 (d, J = 7.2 Hz, 2H), 7.20-7.35 (m, 10H), 7.40 (m, 1H), 7.52 (s, 1H); MS (ion spray) 638 (M+1) ; Anal. Calc'd for C₃₃H₄₃N₅O₈: C, 62.15; H, 6.80; N, 10.98. Found: C, 62.41; H, 6.85; N, 11.09.

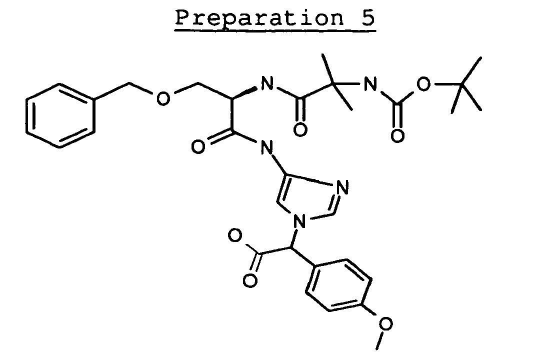

To a solution of the product of Preparation 5A (14.4 g, 23 mmol), stirring in dioxane (150 mL) at room temperature, was added a solution of lithium hydroxide (0.65 g, 27.6 mmol) in water (75 mL). After 20 minutes reaction mixture was acidified to a pH of 2.9 using 1 N hydrochloric acid. To the resulting solution was added water and ethyl acetate. The mixture was then extracted with ethyl acetate. The combined organic extracts were washed with brine, dried over sodium sulfate and concentrated to yield 13.0 g (93%) of the above-identified product as a yellow foam: ¹H NMR (d, DMSO) 1.25-1.40 (m, 15H), 3.65-3.70 (m, 2H), 3.76 (s, 3H), 4.44 (d, J = 3.4 Hz, 2H), 4.57 (m, 1H), 6.20 (s, 1H), 6.97 (d, J = 3.4 Hz, 2H), 7.15-7.35 (m, 10H), 7.42 (m, 1H), 7.53 (s, 1H), 10.2 (s, 1H); MS (ion spray) 610.7 (M+1) ; Anal. Calc'd for C₃₁H₃₉N₅O₈: C, 61.07; H, 6.45; H, 11.49. Found: C, 60.90; H, 6.43; N, 11.32.

### Example 1F

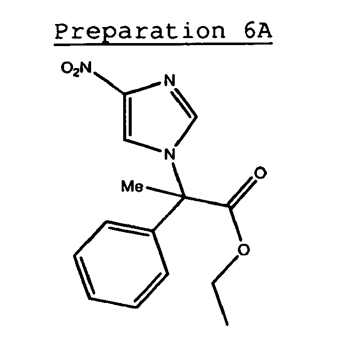

A solution of the product of Preparation 3 (10.00 g, 36.36 mmol) in DMF (50 mL) was added dropwise to a suspension of sodium hydride (1.60 g, 40.00 mmol) in DMF (50 mL) under nitrogen at 0 °C. The mixture was stirred 10 minutes. Ethyl iodide (2.5 mL, 40.00 mmol) was then added dropwise. The reaction mixture was subsequently stirred thirty minutes at 0 °C, and then for 1 hour at ambient temperature. The mixture was quenched with a saturated solution of sodium bicarbonate. Ethyl acetate was added and the mixture washed with bicarbonate followed by brine. The organic layer was dried over sodium sulfate, filtered, and concentrated *in vacuo.* The resulting foam was purified by flash chromatography (300 g silica, 2:3 ethyl acetate/hexanes) to yield the above-identified product (8.81 g, 84%) as a light yellow foam: ¹H NMR (300 MHz, CDCl₃) - consistent with structure; Anal. Calc'd. for C₁₄H₁₅N₃O₄; 58.13 C, 5.23 H, 14.53 N; found 57.88 C, 5.36 H, 14.39 N; FDMS (M+) -289.

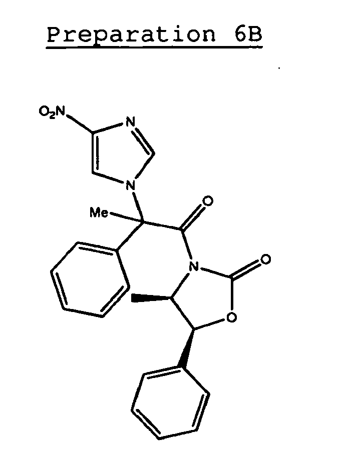

A solution of the product of Preparation 6A (8.35 g, 28.89 mmol) in THF (100 mL) was treated with lithium hydroxide (1.82 g, 43.34 mmol) and water (50 mL). The reaction was stirred at ambient temperature for 30 minutes. Water was added and the mixture washed with diethyl ether. The pH of the aqueous layer was adjusted to 3.0 with 10% sodium bisulfate. The mixture was saturated with sodium chloride and washed with ethyl acetate. The ethyl acetate washes were combined, dried over sodium sulfate, filtered, and concentrated *in vacuo.* The resulting crude solid was dissolved in anhydrous dichloromethane (100 mL) under nitrogen. To this solution was added catalytic DMF (0.1 mL) and excess oxalyl chloride (25 g). This mixture was stirred for 3 hours, then concentrated *in vacuo.* The resulting crude foam was dissolved in THF (20 mL) and added dropwise to a solution of lithium (4R,5S)-(+)-4-methyl-5-phenyl-2-oxazolidinone which was generated by adding n-BuLi (1.6M in hexanes, 19.9 mL, 31.82 mmol) dropwise to a solution of (4R, 5S)-(+)-4-methyl-5-phenyl-2-oxazolidinone (5.64 g, 31.82 mmol) in THF (50 mL) at -78 °C under nitrogen and stirred for 20 minutes; and then used without further purification.

The resulting mixture was stirred at -78 °C for 30 minutes, then warmed to 0 °C. The mixture was quenched with saturated sodium bicarbonate. Ethyl acetate and water were added and the mixture washed with sodium bicarbonate and brine. The organic layer was dried over sodium sulfate, filtered, and concentrated *in vacuo.* The resulting foam was purified by flash chromatography (400 g silica, 5% diethyl ether/dichloromethane) to yield diastereomer 1 (3.76 g, 31% yield) and diastereomer 2 (4.32 g, 36%) of above-identified product as colorless foams:
diastereomer 1 - ¹H NMR (300 MHz, CDCl₃) - consistent with structure; Anal. Calc'd. for C₂₂H₂₀N₄O₅ ; 62.85 C, 4.80 H, 13.33 N; found 60.97 C, 4.64 H, 12.44 N; FDMS (M+) - 420:
diastereomer 2 - ¹H NMR (300 MHz, CDCI₃) - consistent with structure; Anal. Calc'd. for C₂₂H₂₀N₄O₅; 62.85 C, 4.80 H, 13.33 N; found 62.41 C, 4.82 H, 11.92 N; FDMS (M+) - 420.

### Example 1G

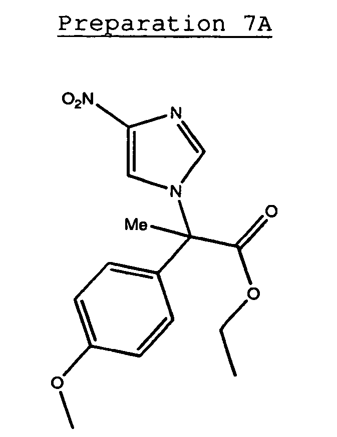

Preparation 7A was prepared, using the method of Preparation 6A, using the product of Preparation 4 (5.00 g, 16.39 mmol) in DMF (25 mL) and sodium hydride (0.72 g, 18.03 mmol) and methyl iodide 1.12 ml, 18.03 mmol) in DMF (25 mL) to yield above-identified product (4.81 g, 92%) as a light yellow foam: ¹H NMR (300 MHz, CDCl₃) - consistent with structure: Anal. calc'd. for C₁₅H₁₇N₃O₅; 56.42 C, 5.37 H, 13.16 N; found 56.13 C, 5.35 H, 13.01 N; ISMS (M+) -320.

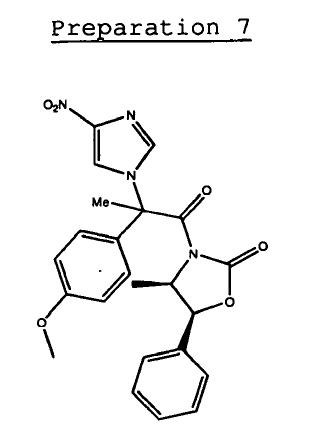

Preparation 7 was prepared, as described in Preparation 6B, using the product of Preparation 7A (4.80 g, 15.03 mmol) in THF (50 mL) and lithium hydroxide (1.26 g, 30.06 mmol) in water (25 mL) to give the crude acid. This material was immediately reacted with anhydrous dichloromethane (100 mL), catalytic DMF (0.5 mL), and excess oxalyl chloride (12 mL) to give the crude acid chloride. This crude product was then reacted with THF (20 mL), n-BuLi (1.6M in hexanes, 14.1 mL, 22.54 mmol), and (4R, 5S)-(+)-4-methyl-5-phenyl-2-oxazolidinone (4.00 g, 22.54 mmol) in THF (50 mL) to yield diastereomer 1 (2.79 g, 41% yield) and diastereomer 2 (2.80 g, 41%) of the above-identified product as colorless foams: 2) diastereomer 1 - ¹H NMR (300 MHz, CDCl₃) - consistent with structure; Anal. calc'd. for C₂₃H₂₂N₄O₆; 61.33 C, 4.92 H, 12.44 N; found 60.92 C, 4.82 H, 12.03 N; ISMS (M+) - 451: ¹H NMR (300 MHz, CDCl₃) - consistent with structure; Anal. calc'd. for C₂₃H₂₂N₄O₆; 61.33 C, 4.92 H, 12.44 N; found 61.57 C, 4.98 H, 12.47 N; ISMS (M+) - 451.

### Example 1I

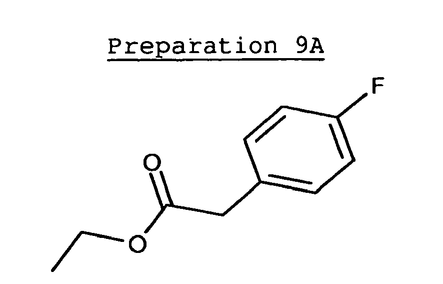

Reaction of 4-fluorophenylacetic acid (15.0 g, 97.0 mmol), p-toluenesulfonic acid (2.0 g, 10.5 mmol) and absolute ethanol (100 mL), as described in Preparation 4A, gave 15.4 g (87%) of the above-identified product as a colorless oil: ¹H-NMR (d, DMSO) 1.17 (t, J = 7.2 Hz, 3H), 3.66 (s, 2H), 4.06 (q, J = 7.2 Hz, 2H), 7.10-7.20 (m, 2H), 7.25-7.35-(m, 2H); MS (FD) 182 (M+); Anal. Calc'd for C₁₀H₁₁FO₂: C, 65.92; H, 6.09. Found: C, 65.67; H, 5.96.

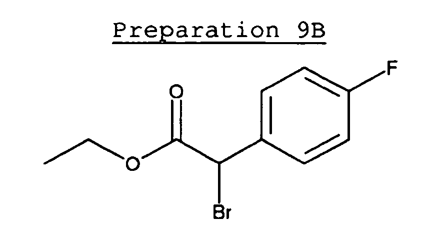

Reaction of the product of Preparation 9A (14.9 g, 82 mmol), N-bromosuccinimide (14.9 g, 84.5 mmol) and 48% HBr (4 drops) in carbon tetrachloride (80 mL) as described in Preparation 4B gave 18.3 g (85%) of the above-identified product, as follows, as a colorless oil: ¹H-NMR (d, DMSO) 1.19 (t, J = 7.2 Hz, 3H), 4.15-4.25 (m, 2H), 5.95 (s, 1H), 7.15-7.30 (m, 2H), 7.56-7.70 (m, 2H); MS (FD) 260, 262 (M+); Anal. Calc'd for C₁₀H₁₀BrFO₂: C, 46.00; H, 3.96. Found: C, 46.10; H, 3.95.

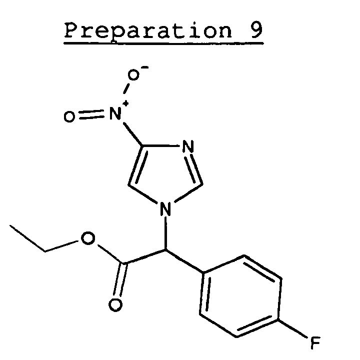

Reaction of the product of Preparation 9B (68 g, 260 mmol), 4-nitroimidazole (35.0 g, 312 mmol) and potassium carbonate (108 g, 780 mmol) in dimethylformamide (300 mL), as described in Preparation 4 gave 39.8 g (52%) of the above-identified product as an orange oil: ¹H-NMR (d, DMSO) 1.83 (t, J = 7.2 Hz, 3H), 4.25 (q, J = 7.2 Hz, 2H), 6.66 (s, 1H), 7.25-7.35 (m, 2H), 7.55-7.65 (m, 2H), 7.95 (d, 1.13 Hz, 1H), 8.44 (d, J = 1.5 Hz, 1H); MS (ion spray) 294.2 (M+1) ; Anal. Calc'd for C₁₃H₁₂FN₃O₄ : C, 53.24; H, 4.12; N, 14.33. Found: C, 53.51; H, 4.07; N, 14.42.

### Example 1J

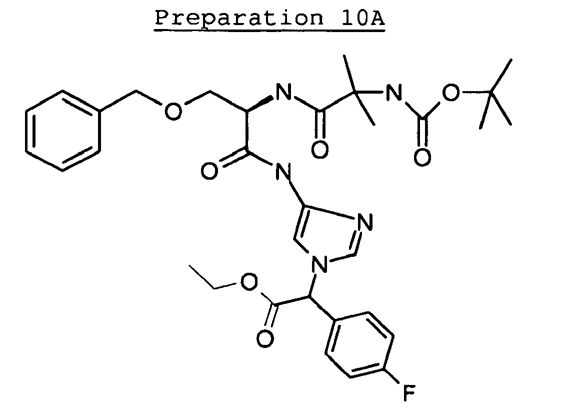

Reduction of the product of Preparation 9 (8.9 g, 30.3 mmol) with 10% palladium on carbon (6.0 g) in tetrahydrofuran (120 mL) followed by coupling with the product of Preparation 1 (11.4 g, 30 mmol), 1-hydroxybenzotriazole (4.5 g, 33 mmol) and 1-(3-dimethylaminopropyl)-3-ethylcarbodiimide (6.8 g, 33 mmol), as described in Preparation 5A, gave 10.8 g (58%) of the above-identified product as a tan foam: ¹H-NMR (d, DMSO) 1.18 (t, J = 7.2 Hz, 3H), 1.25-1.35 (m, 15H), 3.60 (m, 1H), 3.70 (m, 1H), 4.25 (q, J = 7.2 Hz, 2H), 4.44 (d, J = 2.6 Hz, 2H), 4.60 (m, 1H), 6.47 (s, 1H), 7.20-7.40 (m, 9H), 7.40-7.50 (m, 3H), 7.56 (s, 1H), 10.25 (br s, 1H); MS (ion spray) 626.1 (M+1); Anal. Calc'd for C₃₂H₄₀FN₅O₇: C, 61.43; H, 6.44; N, 11.19. Found: C, 61.63; H, 6.42; N, 11.26.

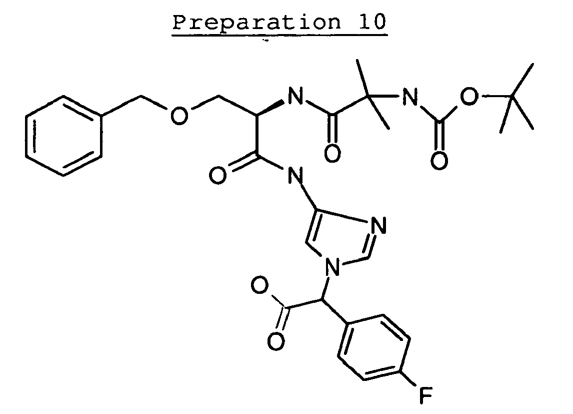

Reaction of the product of Preparation 10A (10.5 g, 17.0 mmol) and lithium hydroxide (0.48 g, 20.4 mmol) in dioxane (200 mL) and water (100 mL) as described in Preparation 5 gave 10.1 g (100%) of the above-identified product as a tan foam: ¹H-NMR (d, DMSO) 1.25-1.40 (m, 15H), 3.35 (br s, 1H), 3.60 (m, 1H), 3.70 (m, 1H), 4.44 (d, J = 2.6 Hz, 2H), 4.60 (m, 1H), 6.33 (s, 1H), 7.20-7. 35 (m, 9H), 7.40-7.50 (m, 3H), 7.56 (s, 1H), 10.20 (br s, 1H); MS (ion spray) 598.5 (M+1) ; Anal. Calc'd for C₃₀H₃₆FN₅O₇: C, 60.29; H, 6.07; N, 11.72. Found: C, 60.38; H, 6.29; N, 11.49.

### Example 1N

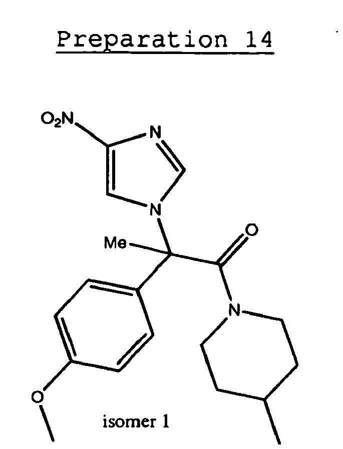

The product of Preparation 7, diastereomer 1 (1.00g, 2.22 mmol) in THF (50 mL) was added to a solution of lithium hydroxide (0.10 g, 2.44 mmol) in water (25 mL). The resulting mixture was stirred at ambient temperature for 30 minutes. Water was added and the mixture washed with diethyl ether. The pH of the aqueous layer was adjusted to 3.0 with 10% aqueous sodium bisulfate. The mixture was saturated with sodium chloride and washed with ethyl acetate. The ethyl acetate washes were combined, dried over sodium sulfate, filtered, and concentrated *in vacuo.* The resulting crude solid was dissolved in anhydrous dichloromethane (50 mL) under nitrogen. To this solution was added catalytic DMF (0.1 mL) and excess oxalyl chloride (5 g). This mixture was stirred 3 hours, then concentrated *in vacuo.*

The resulting crude foam was dissolved in anhydrous dichloromethane (50 mL) and cooled to 0 °C. 4-dimethylaminopyridine (catalytic, 10 mg) and 4-methylpiperidine (0.34 mL, 2.71 mmol) were added and the resulting solution stirred for 18 hours. The resulting crude foam was dissolved in anhydrous dichloromethane (50 mL) washed with sodium bicarbonate and brine. The organic layer was dried over sodium sulfate, filtered, and concentrated *in vacuo.* The crude foam was purified by flash chromatography (silica, 100 g, 5% methanol/dichloromethane) to yield the above-identified product (0.38 g, 50% yield) as a colorless foam: ¹H NMR (300 MHz, CDCl₃) - consistent with structure; Anal. calc'd. for C₁₉H₂₄N₄O₄; 61.28 C, 6.50 H, 15.05 N; found 61.38 C, 6.40 H, 15.11 N; FDMS (M+) - 372.

### Example 1R

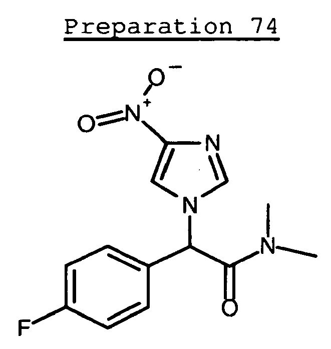

To a solution of the compound of Preparation 9 (17.0 g, 58.0 mmol) stirring at room temperature was added to sodium hydroxide (125 mL of a 2N aqueous solution) along with tetrahydrofuran (10 mL) and ethanol (10 mL). After hydrolysis was complete, the mixture was cooled in an bath and acidified to pH 2.75 with aqueous hydrochloric acid and extracted with ethyl acetate. The combined organic extracts were washed with water, dried over sodium sulfate and concentrated to provide 15.0 g (99%) of the desired carboxylic acid. The crude material was combined with aqueous N,N-dimethyl amine (40%, 9.0 mL, 71.8 mmol), 1-hydroxybenzotriazole hydrate (7.64 g, 56.6 mmol) and 1,3-dicyclohexylcarbodiimide (11.7 g, 56.6 mmol) in tetrahydrofuran (150 mL). After 18 h, the mixture was concentrated and the residue slurried in ethyl acetate, filtered, and the filtrate concentrated. Purification of the concentrate by flash chromatography (silica gel, chloroform/methanol) provided 10.2 g (62%) of the desired product: ESMS: (M+H)⁺ 293.1. ¹H NMR (300 MHz, DMSO-*d*₆) δ 8.21 (d, 1H, J = 1.51 Hz) 7.80(d, 1H, J = 1.13 Hz), 7.60-7.50 (m, 2H,), 7.38-7.25 (m, 2H), 6.88 (s, 1H), 2.92 (s, 3H), 2.86 (s, 3H). *Anal.* Calc'd. for C₁₃H₁₃N₄O₃: C, 53.43; H, 4.48; N, 19.17. Found: C, 53.43; H, 4.71; N, 19.07.

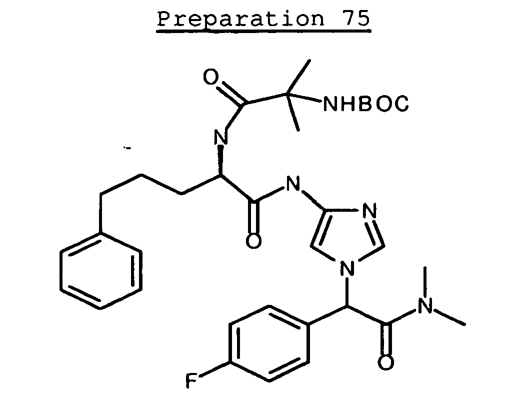

The product of Preparation 74 (2.0 g (6.85 mmol) was combined with 10% palladium/carbon (1.80 g) and palladium/black (0.20 g) in tetrahydrofuran(75 mL)and the mixture shaken under a hydrogen atmosphere (38 psi) in a Parr apparatus. After reduction was complete, the catalyst was removed by filtration through celite and the resulting solution was immediately added to a solution of 1,3-dicyclohexylcarbodiimide (1.51 g, 7.3 mmol), 1-hydroxybenzotriazole (1.0 g, 7.3 mmol), the product of Preparation 2 (2.77 g, 7.3 mmol) in tetrahydrofuran (50 mL) at room temperature. After 16 h, the mixture was concentrated and the residue slurried in ethyl acetate then filtered. The filtrate was concentrated and resulting crude product purified by flash chromatography (silica gel, chloroform/methanol) which afforded 3.47 g (81%) of the desired product: ESMS: (M+H)⁺ 623.5, 624.6. ¹H NMR was consistent with product. *Anal.* Calc' d. for C₃₃H₄₃N₆O₄F.0.02 CHC13: C, 63.44; H, 6.94; N, 13.44. Found: C, 63.04; H, 7.41; N, 11.93.

### Example 1S

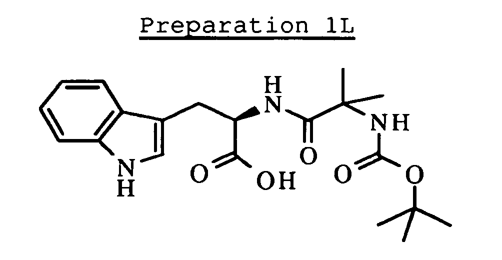

N-Methyl morpholine (4.79 mL, 2 eq, 47.3 mm) was added to a stirred slurry of *N*-Boc-a-aminoisobutyric acid (4.43 g, 21.7 mm, 1 eq) and 3.89 g (21.7 mm, 1.0 eq) of 2-chloro-(4, 6)-dimethoxy-1, 3, 5-triazine (CDMT) in 100 mL of diethyl ether. After stirring the reaction mixture at ambient temperature for 1.5 hours, D-tryptophan ester hydrochloride was added. After stirring overnight, the reaction mixture was quenched by the addition of 150 mL of 10% aqueous citric acid solution. The layers were separated and the ether layer was washed with 50 mL of saturated sodium bicarbonate solution and 50 mL of water. Lithium hydroxide (2.43 g, 5 eq) was dissolved in 100 ml of water and the solution was added to the diethyl ether solution and stirred vigorously for 4 hours at room temperature. The layers were separated and the pH of the aqueous layers was adjusted to 5.6 with 1M HCl. The pH was then adjusted to 3.95 with 10% citric acid solution and the aqueous layer was extracted with 100 mL of ethyl acetate. The ethyl acetate layers were washed with brine, dried over magnesium sulfate and filtered. The volatiles were removed under vacuum to give 82 % yield of the desired product as a white foam. 1H-NMR consistent with structure.

### Preparation R5

### Ethyl 2-[4-((2R)-2-{2-[(Tert-butoxy)carbonylamino]-2-methyl propanoylamino}-3-indol-3-ylpropanoylamino)imidazolyl]-2-phenylacetate

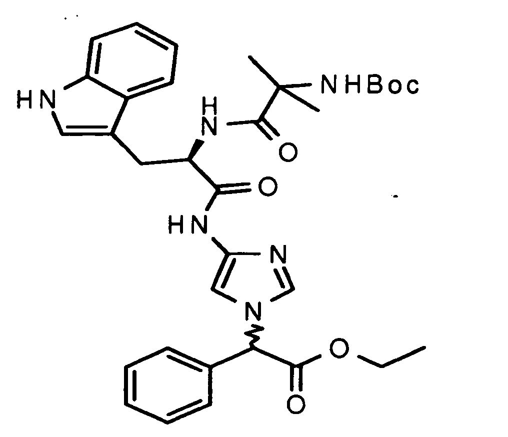

This compound was obtained from the reduction of ethyl 2-(4-nitroimidazolyl)-2-phenylacetate and subsequent reaction with (2R)-2-{2-[(tert-butoxy) carbonylamino]-2-methyl propanoylamino)-3-indole-3-ylpropanoic acid as a yellow foam in 73% yield after purification by flash chromatography using dichloromethane : methanol (19:1) as the eluent. MS (FIA) m/z 617.5 [(M+H)⁺]. ¹H nmr (CDCl₃) : δ 1.19-1.32 (m, 18H), 3.10-3.12 (m, 1H), 3.16-3.17 (m, 1H), 3.32 (s, 1H), 4.22-4.27 (m, 2H), 4.69 (s, broad, 1H), 6.44 (s, 1H), 6.85-6.91 (m, 2H), 7.00 (t, 1H), 7.07-7.08 (m, 1H), 7.38-7.40 (m, 1H), 7.42-7.45 (m, 6H), 7.55-7.56 (m, 2H), 10.16 (s, broad, 1H), 10.75 (s, 1H).

### Example 2

### Example 2-2

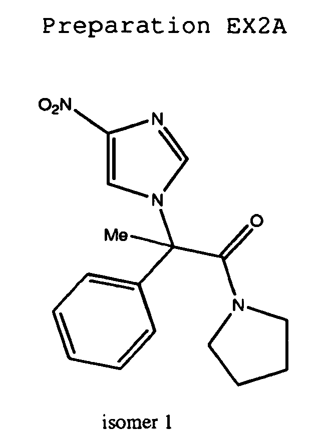

A solution of the product of Preparation 6B, diastereomer 1 (2.30 g, 5.48 mmol) in THF (50 mL) was added to a solution of lithium hydroxide (0.25 g, 6.03 mmol) in water (25 mL). The resulting mixture was stirred at ambient temperature for 30 minutes. Water was added and the mixture washed with diethyl ether. The pH of the aqueous layer was adjusted to 3.0 with 10% aqueous sodium bisulfate. The mixture was saturated with sodium chloride and washed with ethyl acetate. The ethyl acetate washes were combined, dried over sodium sulfate, filtered, and concentrated *in vacuo.* The resulting crude solid was dissolved in anhydrous dichloromethane (50 mL) under nitrogen. To this solution was added catalytic DMF (0.1 mL) and excess oxalyl chloride (5 g). This mixture was stirred for 3 hours, then concentrated *in vacuo.* The resulting crude foam was dissolved in anhydrous dichloromethane (50 mL) and cooled to 0 °C. 4-Dimethylaminopyridine (catalytic, 10 mg) and pyrrolidine (1.8 mL, 18.74 mmol) were added and the resulting solution was stirred for 18 hours. Dichloromethane was then added and the mixture washed with sodium bicarbonate and brine. The organic layer was dried over sodium sulfate, filtered, and concentrated *in vacuo.* The crude foam was purified by flash chromatography (silica, 100 g, 5% methanol/dichloromethane) to yield the above-identified product (1.73 g, 88% yield) as a colorless foam: ¹H NMR (300 MHz, CDCI₃) - consistent with structure; Anal. calc'd. for C₁₆H₁₈N₄O₃; 61.14 C, 5.77 H, 17.82 N; found 60.67 C, 5.78 H, 16.03 N; FDMS (M+) - 314.

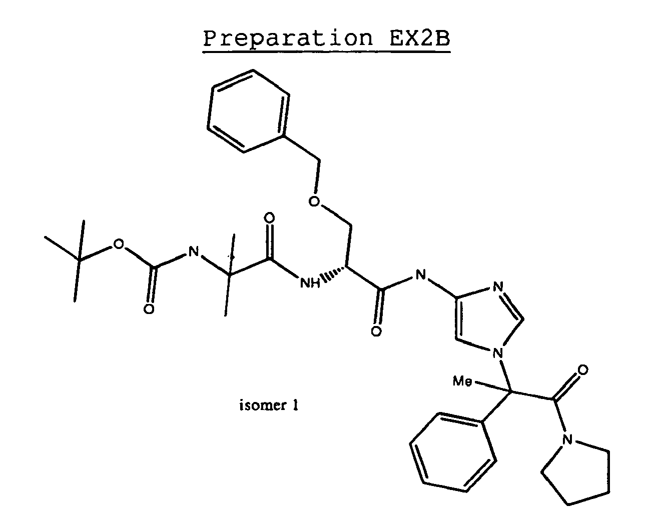

A solution of the product of Preparation EX2A (1.66 g, 5.29 mmol) in THF (5 mL) was added to a suspension of 5% palladium on carbon (0.80 g, catalytic, 25 mL THF) under inert atmosphere. The resulting mixture was placed under hydrogen (40 psi) on a Parr shaker for 1.5 hours. The resulting mixture was placed under nitrogen and celite added. The mixture was then filtered and rinsed with THF. The filtrate was place under nitrogen and HOBT (0.71 g, 5.29 mmol), the product of Preparation 1 (2.01 g, 5.29 mmol), EDC (1.00 g, 5.81 mmol), and DIEA (1.0 mL, 5.81 mmol) were added. The resulting mixture was stirred for 18 hours at ambient temperature, then concentrated *in vacuo.* The crude material was dissolved in ethyl acetate and washed with sodium bicarbonate and brine. The organic layer was dried over sodium sulfate, filtered, and concentrated *in vacuo.* The resulting crude foam was purified by flash chromatography (silica, 100 g, 2% methanol/dichloromethane) to yield the above-identified product (0.66 g, 19% yield) as a light yellow foam: ¹H NMR (300 MHz, CDCl₃) - consistent with structure; Anal. calc'd. for C₃₅H₄₆N₆O₆; 65.00 C, 7.17 H, 12.99 N; found 63.21 C, 6.92 H, 12.54 N; FDMS (M+) - 646.

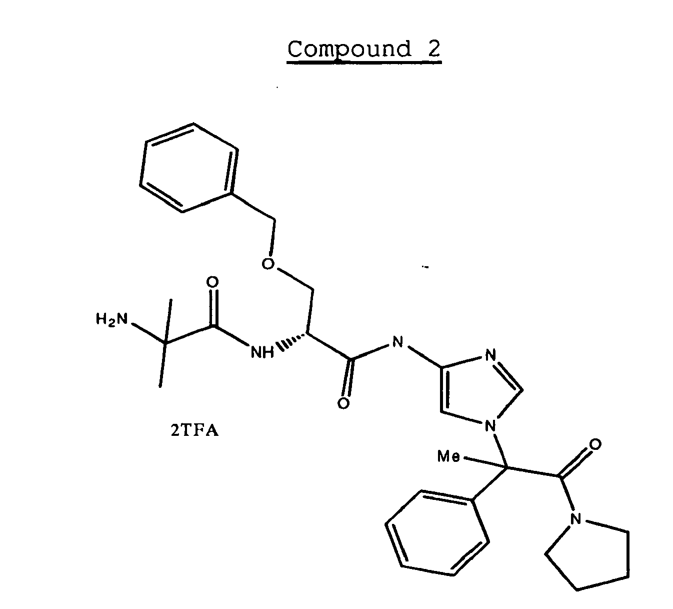

A solution of the product of Preparation EX2B (0.52 g, 0.80 mmol) in dichloromethane (20 mL) was stirred under nitrogen with anisole (0.4 mL) and trifluoroacetic acid (4.0mL) at ambient temperature for 3 hours. The mixture was concentrated *in vacuo* to approximately 5 mL and excess diethyl ether added. The mixture was filtered and rinsed with diethyl ether to yield the above-identified product (0.40 g, 65% yield) as an off white solid: ¹H NMR (300 MHz, CDCl₃) - consistent with structure; Anal. calc'd. for C₃₄H₄₀N₆O₈F₆ ; 52.71 C, 5.20 H, 10.85 N; found 52.60 C, 5.08 H, 10.69 N; FDMS (M+) - 546.

### Example 2-6

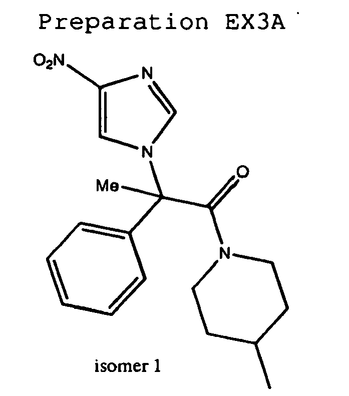

Prepared as in Preparation EX2A using the product of Preparation 6B, diastereomer 1 (1.88 g, 5.44 mmol) in THF (50 mL) and lithium hydroxide (0.23 g, 5.63 mmol) in water (25 mL) to give a crude acid. The resulting crude solid was dissolved in anhydrous dichloromethane (50 mL) and reacted with catalytic DMF (0.1 mL) and excess oxalyl chloride (5 g) to give the crude acid chloride. The resulting crude foam was dissolved in anhydrous dichloromethane (50 mL) and reacted with 4-Dimethylaminopyridine (catalytic, 10 mg), L-proline methyl ester hydrochloride (0.90 g, 5.44 mmol), and N,N-diethylisopropylamine (2.8 mL, 16.31 mmol) to yield the above-identified product (1.21 g, 65% yield) as a colorless foam: ¹H NMR (300 MHz, CDCl₃) - consistent with structure; Anal. calc'd. for C₁₈H₂₂N₄O₃; 63.14 C, 6.48 H, 16.36 N; found 63.29 C, 6.45 H, 15.29 N; FDMS (M+) - 342.

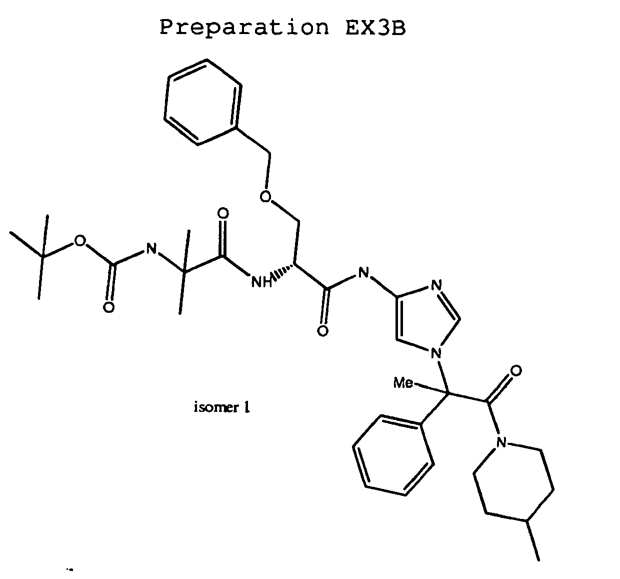

Prepared as in Preparation EX2B using the product of Preparation EX3A (1.21 g, 3.53 mmol) and 5% palladium on carbon (0.80 g, catalytic, 25 mL THF) to give the crude amine. The resulting filtrate was reacted with HOBT (0.48 g, 3.53 mmol), the product of Preparation 1 (1.34 g, 3.53 mmol), diisopropylethylamine (0.6 mL, 3.53 mmol), and EDCI (0.67 g, 3.88 mmol) to yield the above-identified product (0.97 g, 41% yield) as a light yellow foam: ¹H NMR (300 MHz, CDCl₃) - consistent with structure: Anal. calc'd. for C₃₇H₅₀N₆O₆; 65.85 C, 7.47 H, 12.45 N; found 64.96 C, 7.48 H, 12.04; FDMS (M+) - 675.

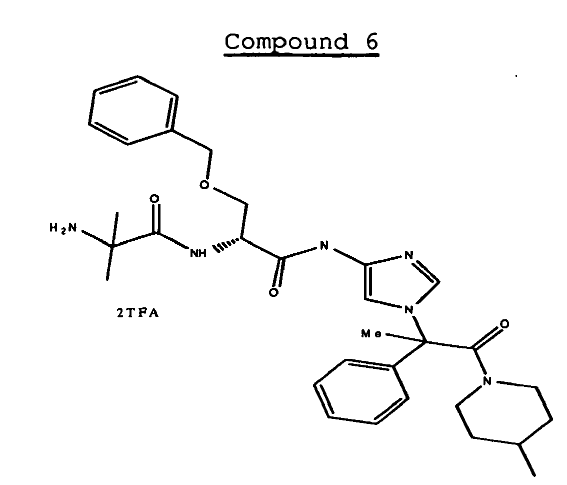

Prepared as in Example 2-2 using the product of Preparation EX3B (0.95 g, 1.41 mmol), trifluoroacetic acid (4.0 mL), anisole (0.4 mL), and dichloromethane (20 mL) to yield the desired product (Preparation 3) (0.82 g, 92%) as a pale yellow solid: ¹H NMR (300 MHz, CDCl₃) consistent with structure above: Anal. calc'd. for C₃₆H₄₄N₆O₈F₆ ; 53.86 C, 5.53 H, 10.47 N; found 52.73 C, 5.50 H, 10.07 N; FDMS (M+) - 574.

### Example 2-7

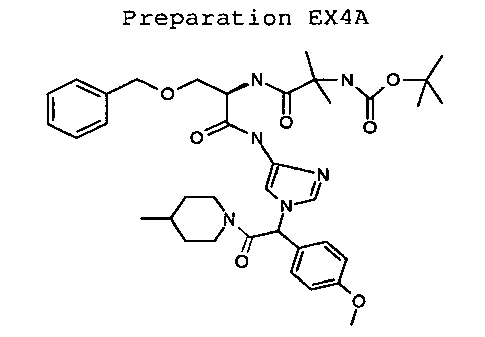

To a solution of the product of Preparation 5 (8.0 g,13.0 mmol), stirring in dimethylformamide (150 mL) at room temperature, was added 4-methylpiperidine (1.6 mL, 13.0 mmol), 1-hydroxybenzotriazole (2.0 g, 14.3 mmol) and 1-(3-dimethylaminopropyl)-3-ethylcarbodiimide (3.0 g, 14.3 mmol). After 16 hours, the reaction mixture was filtered and concentrated. The resulting material was partitioned between ethyl acetate and water and extracted with ethyl acetate. The combined organic extracts were washed with brine, dried over sodium sulfate, filtered and concentrated to dryness. The resulting crude material was purified by flash chromatography (silica gel, 3% methanol/ chloroform) to yield 7.65 g (85%) of the above-identified product as a yellow foam: ¹H-NMR (d, DMSO) 0.2 (m, 1H), 0.50 (d, J = 6.0 Hz, 1.5 H), 0.80 (d, J = 6.0 Hz, 1.5 H), 1.05 (m, 1H), 1.22-1.45 (m, 15H), 1.50-1.65 (m, 4H), 2.65 (m, 1H), 3.00 (m, 1H), 3.55 (m, 1H), 3.65 (m, 1H), 3.75 (s, 3H), 4.37 (m, 1H), 4.40-4.50 (m, 2H), 4.60 (m, 1H), 6.62 (d, J = 13 Hz, 1H), 6.98 (t, J = 9.4 Hz, 2H), 7.10-7.45 (m, 11H), 10.15 (br s, 1H); MS (ion spray) 691.3 (M+1) ; Anal. Calc'd for C₃₇H₅₀N₆O₇·0.6H₂O: C, 63.34; H, 7.35; N, 11.98. Found: C, 63.25; H, 7.03; 11.87.

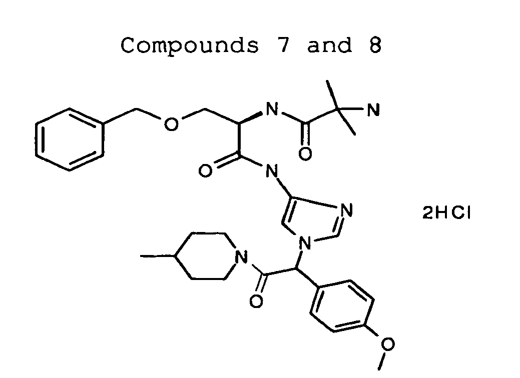

To a solution of the product of Preparation EX4A (7.26 g, 10.5 mmol), stirring in dichloromethane (25 mL) at room temperature, was added trifluoroacetic acid (10 mL). After 4 hours, the reaction mixture was poured into a saturated solution of sodium bicarbonate extracted with chloroform. The combined organic extracts were washed with brine, dried over sodium sulfate, filtered and concentrated to yield 6.12 g (99%) of the free base as a tan foam. The diastereomeric material (3.0 g) was chromatographed on an 8 x 15 cm Prochrom column packed with Kromasil CHI-DMP chiral phase using an eluent mixture of 3A alcohol (13% by v), dimethylethylamine (0.2% by v) in heptane at a flow rate of 250 mL/min to provide the individual diastereomers in pure form:

Compound 7 Isomer: To a solution of the purified isomer in ethyl acetate was added a saturated solution of hydrochloric acid in diethyl ether. The resulting slurry was concentrated to dryness to yield 1.1 g (37%) of the desired product as a white solid: ¹H NMR (d, DMSO) 0.50 (d, J = 6.0 Hz, 1.5 H), 0.80 (d, J = 6.0 Hz, 1.5 H), 1.16 (m, 1H), 1.35 (m, 1H), 1.50-1.70 (m, 8H), 2.60-2.70 (m, 2H), 3.03 (m, 1H), 3.65-3.80 (m, 6H), 4.40 (m, 1H), 4.53 (s, 2H), 4.75 (m, 1H), 6.90-7.08 (m, 3H), 7.25-7.45 (m, 9H), 8.20-8.40 (m, 4H), 8.61 (d, J = 7.5 Hz, 1H), 11.15 (br s, 1H); t_{R} = 7.93 min; MS (ion spray) 591.6 (M+1); Anal. Calc'd for C₃₂H₄₂N₆O₅·2HCl: C, 57.92; H, 6.69; N, 12.66. Found: C, 57.72; H, 6.47; N, 12.42.

Compound 8 Isomer: To a solution of the purified isomer in ethyl acetate was added a saturated solution of hydrochloric acid in diethyl ether. The resulting slurry was concentrated to yield 0.98 g (33%) of the desired product as a white solid: ¹H NMR (d, DMSO) 0.50 (d, J = 6.0 Hz, 1.5 H), 0.80 (d, J = 6.0 Hz, 1.5 H), 1.16 (m, 1H), 1.35 (m, 1H), 1.50-1.70 (m, 8H), 2.60-2.70 (m, 2H), 3.03 (m, 1H), 3.65-3.80 (m, 6H), 4.40 (m, 1H), 4.53 (s, 2H), 4.75 (m, 1H), 6.90-7.08 (m, 3H), 7.25-7.45 (m, 9H), 8.20-8.40 (m, 4H), 8.61 (d, J = 7.5 Hz, 1H), 11.15 (br s, 1H) ; t_{R} = 11.78 min; MS (ion spray) 591.6 (M+1) ; Anal. Calc'd for C₃₂H₄₂N₆O₅·2.2HCl : C, 57.29; H, 6.64; N, 12.53. Found: C, 57.23; H, 6.29; N, 12.57.

### Example 2-8

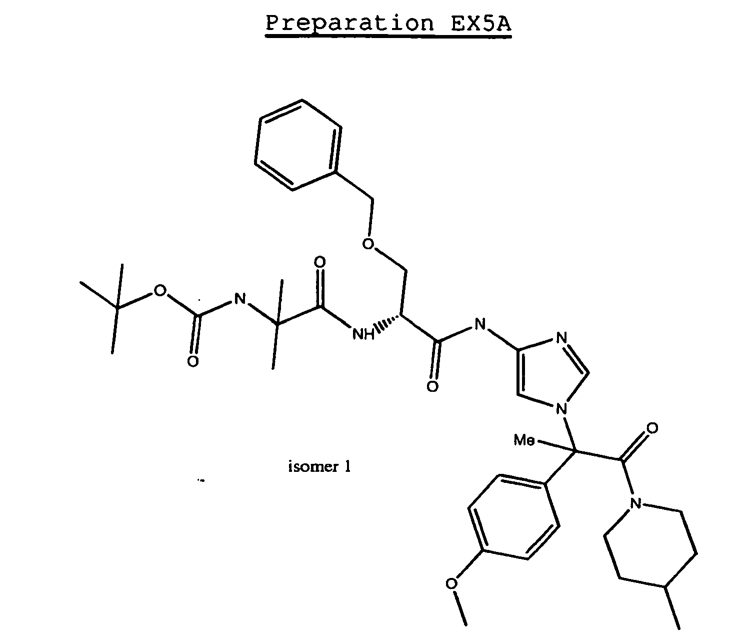

Preparation EX5A was prepared as in Preparation EX2B using the product of Preparation 14 (1.32 g, 3.55 mmol) and 5% palladium on carbon (1.4 g, catalytic, 50 mL THF) to give the crude amine. The resulting filtrate was reacted with HOBT (0.48 g, 3.55 mmol), the product of Preparation 1 (1.35 g, 3.55 mmol), and DCC (0.81 g, 3.91 mmol) to yield the above-identified product (0.82 g, 33% yield), as follows, as a light yellow foam: ¹H NMR (300 MHz, CDCl₃) - consistent with the structure; Anal. calc'd. for C₃₈H₅₂N₆O₇; 64.75 C, 7.44 H, 11.92 N; found 66.19 C, 7.17 H, 12.10 N; ISMS (M+) - 705.

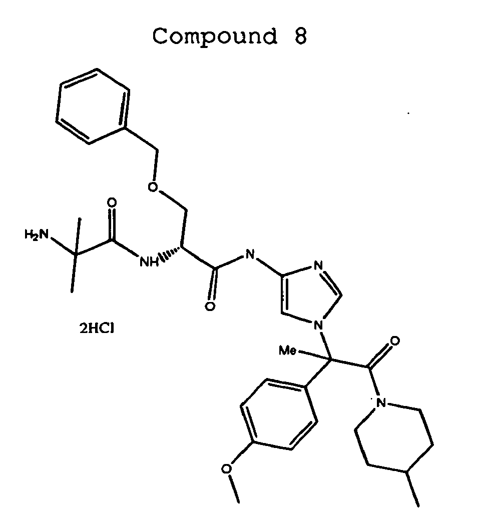

A solution of the product of Preparation EX5A (0.82g, 1.16 mmol), in dichloromethane (20 mL), was stirred under nitrogen with anisole (0.4 mL) and trifluoroacetic acid at ambient temperature for 3 hours. The mixture was quenched with saturated sodium bicarbonate and stirred for 10 minutes at ambient temperature. Dichloromethane was added and the mixture was washed with bicarbonate and brine. The organic layer was dried over sodium sulfate, concentrated in vacuo, and redissolved in 2 mL ethyl acetate. Diethyl ether (saturated HC1(g), 5 mL) was added and the mixture was then stirred for 12 minutes. The mixture was filtered to yield the above-identified product (0.71 g, 90%) as a pale yellow solid: ¹H NMR (300 MHz, CDCl₃) - consistent with structure; Anal. calc'd. for C₃₃H₄₆N₆O₅Cl₂ ; 58.49 C, 6.84 H, 12.40 N; found 55.40 C, 6.48 H, 11.80 N; ISMS (M+) - 605.

### Example 2-9

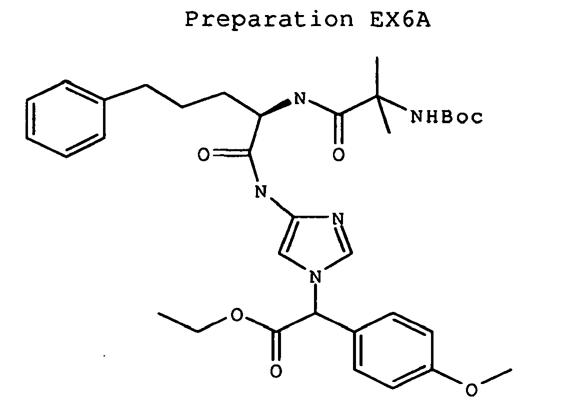

To a suspension of 5% palladium on carbon (1.75 g) and tetrahydrofuran (120 mL) was added the product of Preparation 4 (3.51 g, 11.5 mmol). The reaction mixture was placed under a hydrogen atmosphere (40 mm Hg) on a Parr apparatus for 2 hours then filtered through celite. The filtrate was subsequently added to a solution of the product of Preparation 2 (4.33 g, 11.5 mmol), 1-(3-dimethylaminopropyl)-3-ethylcarbodiimide (2.60 g, 12.6 mmol) and 1-hydroxybenzotriazole (1.72 g, 12.6 mmol) stirring in tetrahydrofuran (50 mL) at 0 °C. After 16 hours at room temperature, the reaction mixture was concentrated. The resulting residue was dissolved in ethyl acetate, filtered and the resulting filtrate concentrated. The crude residue was purified by flash chromatography (silica gel, 90 % ethyl acetate/hexanes to 10 % methanol/ethyl acetate gradient) to give 4.5 g (62 %) the desired product (Preparation EX6A), as follows, as a light orange foam: ¹H NMR consistent with structure; MS (IS) m/e 636 (M + 1). Anal. (C₃₄H₄₅N₅O₇) C, H, N.

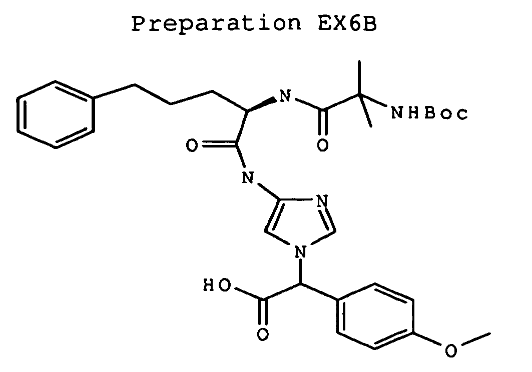

To a solution of the product of Preparation EX6A (1.01 g, 1.59 mmol), stirring in tetrahydrofuran (30 mL) and water (15 mL) at room temperature, was added lithium hydroxide (0.26 g, 6.30 mmol). After 25 minutes, the reaction mixture was concentrated and the resulting residue was diluted with water and extracted with diethyl ether. The aqueous extracts were acidified to pH 2-3 with 1N hydrochloric acid and then extracted with ethyl acetate. The combined organic extracts were washed with brine, dried with sodium sulfate and concentrated to provide 0.96 g (99 %) of the desired compound (Preparation EX6B), as follows, as a light tan foam that was used without further purification: ¹H NMR consistent with structure; MS (IS) m/e 608 (M + 1). Anal. (C₃₂H₄₁N₅O₇) C: calc'd, 63.25; found, 62.68, H, N.

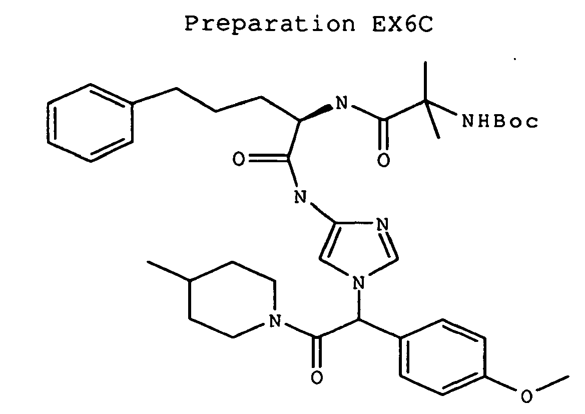

To a solution of the product of Preparation EX6B (0.93 g, 1.53 mmol), stirring in dichloromethane (25 mL) at room temperature, was added N-methylmorpholine (0.20 mL, 1.83 mmol) and 2-chloro-(4,6)-dimethoxy-1,3,5-triazine (0.35 g, 1.99 mmol). After 1 hour, 4-methylpiperidine (0.20 mL, 1.68 mmol) was added and the resulting mixture was stirred room temperature for 2 hours at which time 2-chloro-(4,6)-dimethoxy-1,3,5-triazine (0.10 g, 0.70 mmol) was added. After 1 hour, the reaction mixture was concentrated and the resulting residue purified by flash chromatography (silica gel, ethyl acetate/methanol gradient) to give the desired compound (Preparation EX6C), as follows, as a light yellow solid foam (0.875 g, 83%) : ¹H NMR consistent with structure; MS (IS) m/e 689 (M + 1). Anal.(C₃₈H₅₂N₆O₆) C,H,N.

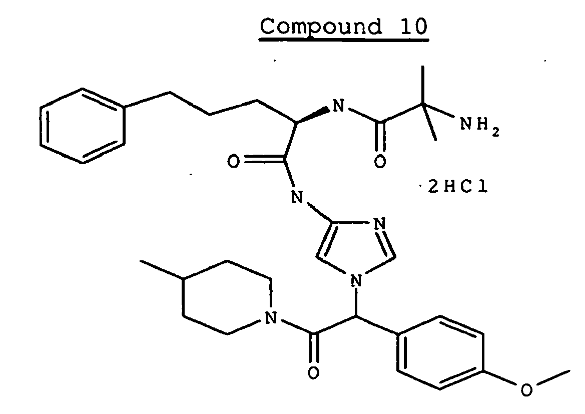

To a solution of the product of Preparation EX6C (0.77 g, 1.12 mmol) and anisole (0.13 mL, 1.13 mmol) stirring in dichloromethane (20 mL) at 0 °C, was added trifluoroacetic acid. After 3-4 hours, the reaction mixture was warmed to room temperature and then quenched by pouring over cold saturated aqueous sodium bicarbonate. The organic layer was collected and the aqueous layer was extracted twice with dichloromethane. The combined organic extracts were washed with aqueous sodium bicarbonate, water, brine, then dried over sodium sulfate and concentrated. The resulting material was purified by flash chromatography (silica gel, 5% methanol/ 95% ethyl acetate gradient to 5% triethylamine/10% methanol/ 85% ethyl acetate) to provide 0.63 g (95 %) of the desired mixture of diastereomers as an off-white solid foam. The mixture (190 mg) was resolved by chiral HPLC [Kromasil packing material, 15% 3A alcohol/ 85% heptane (w/ 0.2% dimethylamine)] to provide the two desired diastereomers. To a solution of diastereomer 2 (65 mg) (retention time = 9.00 min) stirring in ethyl acetate (5 mL) was added saturated solution of hydrochloric acid in diethyl ether. The resulting white precipitate was collected by vacuum filtration and rinsed with diethyl ether to provide the desired compound (60 mg) as a white amorphous solid: ¹H NMR consistent with structure; MS (IS) *m*/*e* 589 (M + 1). Anal. (C₃₃H₄₄N₆O₄·2HCl) C, H, N.

### Example 2-10

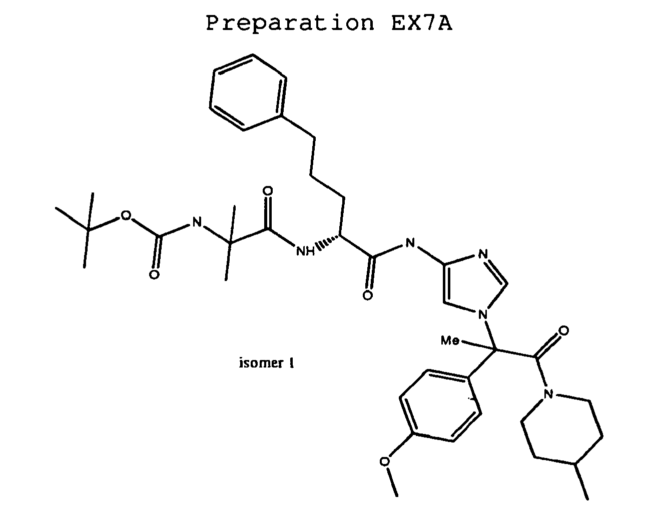

Prepared as in Preparation EX2B using the product of Preparation 14 (0.92 g, 2.47 mmol) and 5% palladium on carbon (1.00 g, catalytic, 30 mL THF) to give the crude amine. The resulting filtrate was reacted with HOBT (0.35 g, 2.47 mmol), the product of Preparation 2 (0.94 g, 2.47 mmol), and DCC (0.56 g, 2.72 mmol) to yield the desired product (Preparation EX7A), as follows, (0.92 g, 53% yield) as a light yellow foam: ¹H NMR (300 MHz, CDCl₃) - consistent with structure; Anal. calc'd. for C₃₉H₅₄N₆O₆; 66.64 C, 7.74 H, 11.96 N; found 66.65 C, 7.65 H, 12.02 N; ISMS (M+) - 702.

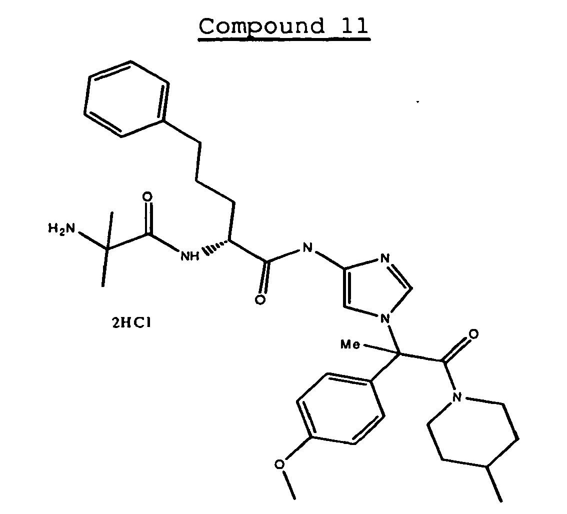

Prepared as in Example 2-8 using the product of Preparation-EX7A (0.26 g, 0.37 mmol), trifluoroacetic acid (4.0 mL), anisole (0.4 mL), and dichloromethane (20 mL) to yield the desired product (Example 7) (0.19 g, 76%) as a pale yellow solid: ¹H NMR (300 MHz, CDCI₃) - consistent with structure; Anal. calc'd. for C₃₄H₄₈N₆O₄Cl₂ ; 60.44 C, 7.16 H, 12.44 N; found 60.08 C, 7.03 H, 12.06 N; ISMS (M+) - 603.

### Example 2-21

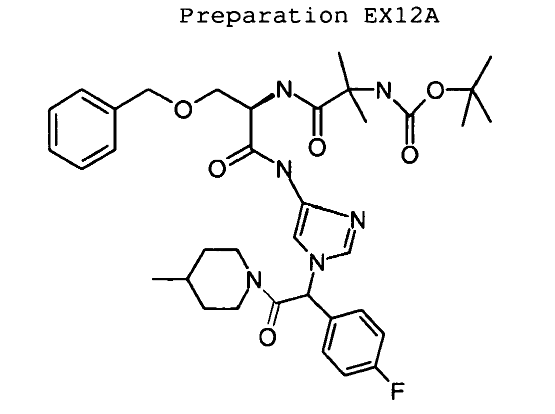

Reaction of product of Preparation 10 (9.2 g, 15.4 mmol), 4-methylpiperidine (1.83 mL, 15.4 mmol), 1-hydroxybenzotriazole (2.3 g, 17 mmol) and 1-(3-dimethylaminopropyl)-3-ethylcarbodiimide (3.5 g, 17 mmol) in dimethylformamide (100 mL) as described in Preparation EX4A gave 9.7 g (93%) of the desired product (Preparation EX12A), as follows, as a tan foam: ¹H-NMR (d, DMSO) 0.76 (d, J = 6.1 Hz, 1.5H), 0.86 (d, J = 6.1 Hz, 1.5H), 1.00 (m, 1H), 1.20-1.40 (m, 15H), 1.45-1.70 (m, 3H), 2.55-2.70 (m, 2H), 3.05 (m, 1H), 3.60 (m, 1H), 3.65-3.75 (m, 2H), 4.40 (m, 1H), 4.44 (d, J = 2.6 Hz, 2H), 4.60 (m, 1H), 6.73 (d, J = 11.3 Hz, 1H), 7.15-7.35 (m, 9H), 7.35-7.50 (m, 4H), 10.20 (br s, 1H). MS (ion spray) 679.6 (M+1) ; Anal. Calc'd for C₃₆H₄₇FN₆O₆: C, 63.70; H, 6.98; N, 12.38. Found: C, 63.44; H, 6.86; N, 12.22.

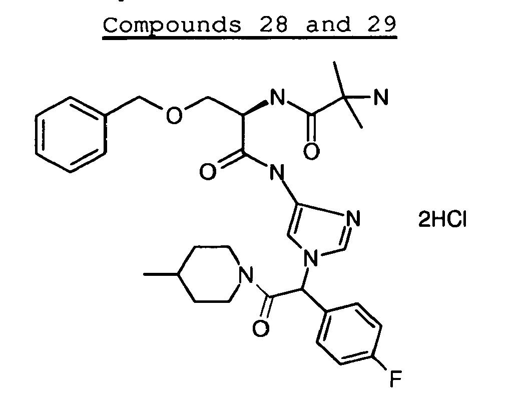

Reaction of the product of Preparation EX12A (9.7 g, 14.3 mmol) with trifluoroacetic acid (16 mL) in dichloromethane (40 mL), as described in Example 2-7, gave 6.8 g (73%) of the desired product (Example 12) as a mixture of diastereoisomers. The mixture (3.2 g) was purified by HPLC (8 x 15 cm Prochrom column packed with Kromasil CHI-DMP chiral phase with an eluent mixture of 3A alcohol and dimethylethylamine in heptane) to give 0.8 g (24 %) of isomer 1 and 0.9 g (26 %) of isomer 2 as white solids:

Compound 28 (Isomer 1). ¹H-NMR (d, DMSO) 0.75 (d, J = 6.4 Hz, 1.5H), 0.88 (d, J = 6.4 Hz, 1.5H), 1.10 (m, 1H), 1.35 (m, 1H), 1.45-1.70 (m, 8H), 2.60-2.75 (m, 2H), 3.15 (m, 1H), 3.65-3.85 (m, 3H), 4.35 (m, 1H), 4.52 (s, 2H), 4.75 (m, 1H), 6.95 (d, J = 11.3 Hz, 1H), 7.20-7.49 (m, 9H), 7.45 (m, 1H), 7.52 (m, 1H), 8.05 (br s, 1H), 8.25 (m, 3H), 8.56 (m, 1H), 10.95 (br s, 1H) ; t_{R} = 6.73 min; MS (ion spray) 579.4 (M+1) ; Anal. Calc'd for C₃₁H₃₉FN₆O₄·2HCl.2CHCl3 : C, 56.29; H, 6.24; N, 12.67. Found: C, 56.47; H, 6.17; N, 12.24.

Compound 29 (Isomer 2) ¹H-NMR (d, DMSO) 0.75 (d, J = 6.4 Hz, 1.5H), 0.88 (d, J = 6.4 Hz, 1.5H), 1.10 (m, 1H), 1.35 (m, 1H), 1.45-1.70 (m, 8H), 2.60-2.75 (m, 2H), 3.15 (m, 1H), 3.65-3.85 (m, 3H), 4.35 (m, 1H), 4.52 (s, 2H), 4.75 (m, 1H), 6.95 (d, J = 11.3 Hz, 1H), 7.20-7.49 (m, 9H), 7.45 (m, 1H), 7.52 (m, 1H), 8.05 (br s, 1H), 8.25 (m, 3H), 8.56 (m, 1H), 10.95 (br s, 1H); t_{R} = 9.09 min; MS (ion spray) 579.4 (M+1) ; Anal. Calc'd for C₃₁H₃₉FN₆O₄·2HCl: C, 57.14; H, 6.34; N, 12.90. Found: C, 57.17; H, 6.18; N, 12.79.

### Example 2-22

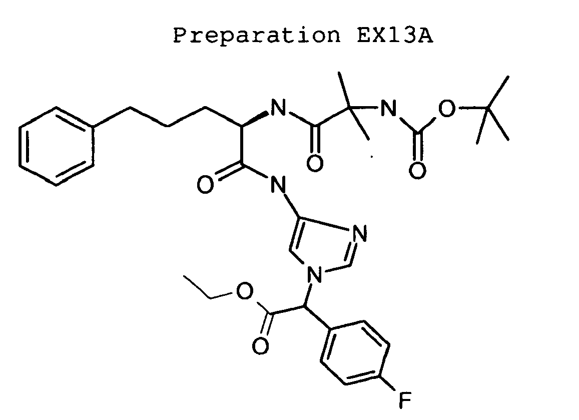

Reduction of the product of Preparation 9 (4.8 g, 16.0 mmol) with 10% palladium on carbon (5.0 g) and tetrahydrofuran (160 mL) followed by coupling with the product of Preparation 2 (6.0 g, 16.0 mmol), 1-hydroxybenzotriazole (2.4 g, 17.6 mmol) and 1-(3-dimethylaminopropyl)-3-ethylcarbodiimide (3.6 g, 17.6 mmol) as described in Preparation 5A gave 15.4 g (77%) of the above-identified product as a tan foam: 1H-NMR (d, DMSO) 1.17 (t, J = 7.2 Hz, 3H), 1.23-1.45 (m, 15H), 1.45-1.57 (m, 6H), 7.16 (q, J = 6.8 Hz, 2H), 4.40 (m, 1H), 6.45 (s, 1H), 7.05 (m, 1H), 7.10-7.30 (m, 8H), 7.40-7.48 (m, 3H), 7.54 (s, 1H), 10.20 (br s, 1H); MS (ion spray) 624.4 (M+1) ; Anal. Calc'd for C₃₃H₄₂FN₅O₆: C, 63.55; H, 6.79; N, 11.23. Found: C, 63.83; H, 6.78; N, 11.38.

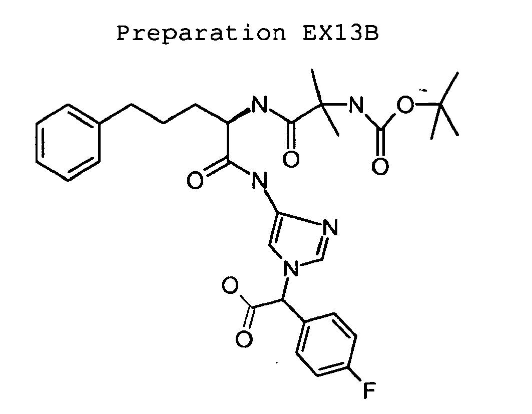

Reaction of the product of Preparation EX 13A (14.8 g, 24.0 mmol) with lithium hydroxide (0.66 g, 29.0 mmol) in dioxane (200 mL) and water (100 mL) as in described in Preparation 5 gave 14.3 g (100%) of the above-identified product as a tan foam: ¹H-NMR (d, DMSO) 1.25-1.40 (m, 15H), 1.50-1.75 (m, 6H), 4.40 (s, 1H), 6.60 (s, 1H), 7.05 (s, 1H), 7.10-7.30 (m, 8H), 7.40-7.50 (m, 3H), 7.55 (s, 1H), 10.2 (br s, 1H), 13.63 (br s, 1H); MS (ion spray) 596.5 (M+1); Anal. Calc'd for C₃₁H₃₈FN₅O₆·0.1dioxane: C, 62.39; H, 6.47; N, 11.59. Found: C, 62.16; H, 6.56; N, 11.28.

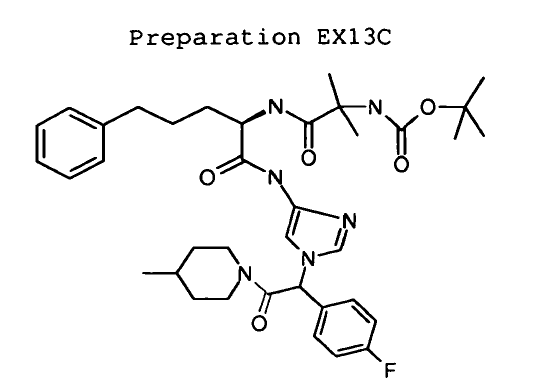

Reaction of the product of Preparation EX13B (13.3 g, 23.1 mmol), 4-methylpiperidine (3 mL, 23.1 mmol), 1-hydroxybenzotriazole (3.4 g, 25.4 mmol) and 1-(3-dimethylaminopropyl)-3-ethylcarbodiimide (5.2 g, 25.4 mmol) in dimethylformamide (100 mL), as described in Preparation EX4A, gave 14.4 g (93%) of the above-identified product as a tan foam: ¹H-NMR (d, DMSO) 0.76 (d, J = 6.4 Hz, 1.5 H), 0.86 (d, J = 4.9 Hz, 1.5H), 1.00 (m, 1H), 1.25-1.45 (m, 17H), 1.45-1.75 (m, 8H), 2.60-2.80 (m, 2H), 3.75 (m, 1H), 4.30-4.45 (m, 2H), 6.71 (d J = 11.7 Hz, 1H), 7.05 (m, 1H), 7.10-7.30 (m, 9H), 7.30-7.45 (m, 3H), 10.15 (m, 1H); MS (ion spray) 677.5 (M+1) ; Anal. Calc'd for C₃₇H₄₉FN₆O₅: C, 65.66; H, 7.30; N, 12.42. Found: C, 65.78; H, 7.19; N, 12.44.

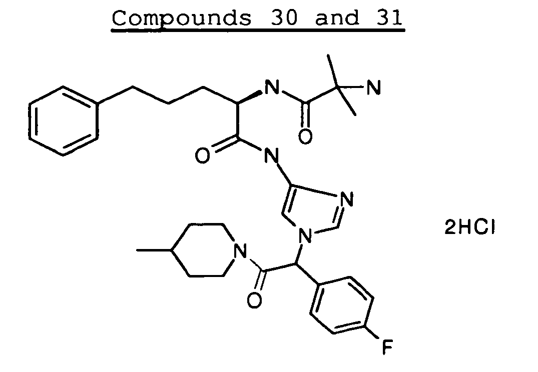

Reaction of the product of Preparation EX13C (13.8 g, 20.4 mmol) with trifluoroacetic acid (16 mL) in dichloromethane (40 mL), as described in Example 2-7 gave 10.5 g (89%) of the desired mixture (Example 13) as a tan foam. The mixture (4.0 g) was purified by HPLC (8 x 15 cm Prochrom column packed with Kromasil CHI-DMP chiral phase with an eluent mixture of 3A alcohol and dimethylethylamine in heptane) to give 1.5 g (38 %) of isomer 1 and 0.77 g (20%) of isomer 2 as white solids:

Compound 30 (isomer 1) ¹H-NMR (d, DMSO) 0.75 (t, J = 6.4 Hz, 1.5 H), 0.87 (t, J = 6.0 Hz, 1.5 H), 1.15 (m, 1H), 1.35 (m, 1H), 1.45-1.80 (m, 12H), 2.55-2.75 (m, 3H), 3.05 (m, 1H), 3.65-3.75 (m, 2H), 4.30-4.50 (m, 2H), 6.94 (d, J = 12 Hz, 1H), 7.10-7.20 (m, 2H), 7.20-7.40 (m, 7H), 7.45 (m, 1H), 7.55 (m, 1H), 8.08 (m, 1H), 8.15-8.30 (m, 3H), 8.44 (t, J = 7.2 Hz, 1H), 10.90 (br s, 1H); t_{R} = 6.62 min; MS (ion spray) 578.3 (M+1); Anal. Calc'd for C₃₂H₄₁FN₆O₃·2.3HCl: C, 58.81; H, 6.61; N, 12.72. Found: C, 57.91; H, 6.55; N, 12.72.

Compound 31 (isomer 2) ¹H-NMR (d, DMSO) 0.75 (t, J = 6.4 Hz, 1.5 H), 0.87 (t, J = 6.0 Hz, 1.5 H), 1.15 (m, 1H), 1.35 (m, 1H), 1.45-1.80 (m, 12H), 2.55-2.75 (m, 3H), 3.05 (m, 1H), 3.65-3.75 (m, 2H), 4.30-4.50 (m, 2H), 6.94 (d, J = 12 Hz, 1H), 7.10-7.20 (m, 2H), 7.20-7.40 (m, 7H), 7.45 (m, 1H), 7.55 (m, 1H), 8.08 (m, 1H), 8.15-8.30 (m, 3H), 8.44 (t, J = 7.2 Hz, 1H), 10.90 (br s, 1H) ; t_{R} = 8.95 min; MS (ion spray) 578.3 (M+1) ; Anal. Calc'd for C₃₂H₄₁FN₆O₃·2.3HCl: C, 58.81; H, 6.61; N, 12.72. Found: C, 58.05; H, 6.64; N, 12.43.

### Example 2-23

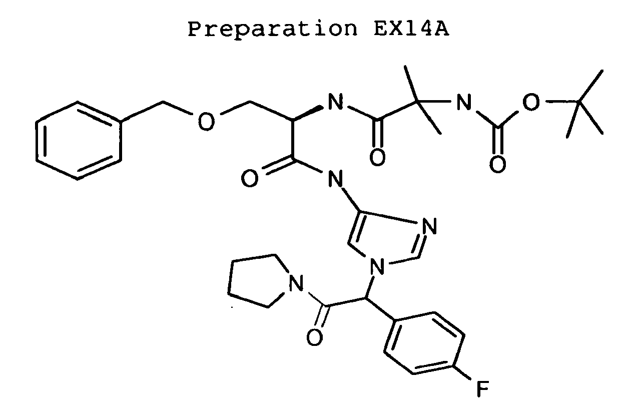

Reaction of the product of Preparation 10B (0.6 g, 1.0 mmol), pyrrolidine (0.08 mL, 1.0 mmol), 1-hydroxybenzotriazole (0.15 g, 1.1 mmol) and 1-(3-dimethylaminopropyl)-3-ethylcarbodiimide (0.23 g, 1.1 mmol) in dimethylformamide (20 mL) as described in Preparation EX4A gave 0.27 g (41%) of the above-identified product as a white foam: ¹H-NMR is consistent with structure; MS (FD) 650.5 (M+) ; Anal. Calc'd for C₃₄H₄₃FN₆O₆·0.6H₂0: C, 61.73; H, 6.73; N, 1212.70. Found: C, 61.98; H, 6.43; N, 12.66.

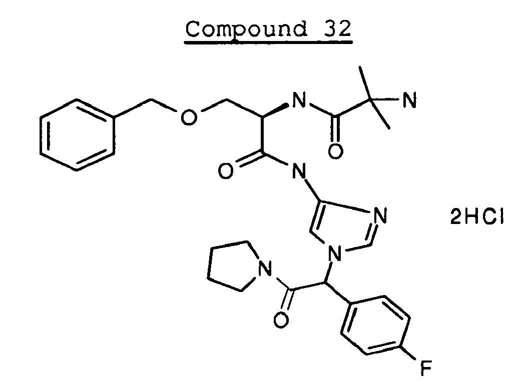

Reaction of the product of Preparation EX14A (0.2 g, 0.3 mmol) and trifluoroacetic acid (4 mL) in dichloromethane (6 mL), as described in Example 2-7, gave 0.16 g (84%) of the desired mixture of isomers as a yellow solid: ¹H-NMR is consistent with structure. MS (high res) calc'd for C₂₉H₃₆FN₆O₄: 551.2782. Found: 551.2790.

### Example 2-24

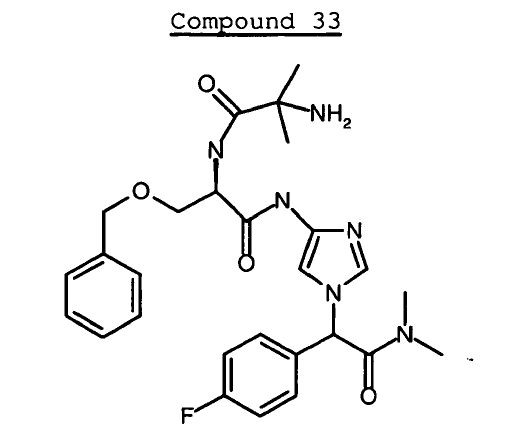

To a solution of the product of Preparation 75 (3.30 g, 5.3 mmol) stirring in dichloromethane (30 mL) at room temperature was added trifluoroacetic acid (10 m). After 3 h, the mixture was concentrated and the residue treated with excess aqueous sodium bicarbonate. The resulting mixture was extracted with ethyl acetate and the combined organic extracts were washed with 1N aqueous sodium hydroxide, dried over sodium sulfate, and concentrated. The residue was purified by flash chromatography (silica gel, chloroform/methanol) to provide 1.40 g (51%) of the desired product as a light tan solid: ESMS: (M+H)⁺ 525.3. ¹H NMR was consistent with product. *Anal.* Calc'd. for C₂₇H₃₃N₆O₄F 1.3 methanol: C, 60.03; H, 6.80; N, 14.84. Found: C, 60.19; H, 6.81; N, 14.56. The isomeric mixture (3.20 g) was separated as previously described in Example 2-9 to give 1.57 g of isomer 1 (t_{R} = 7.57 min) and 0.88 g of isomer 2 (t_{R} = 10.43 min). For isomer 2, 0.88 g (1.68 mmol) was dissolved in ethyl acetate and treated with a saturated solution of hydrochloric acid in diethyl ether. The resulting mixture was concentrated, washed with diethyl ether to give 0.97 g of the desired product: ESMS: (M+H)⁺ 525.4, 526.7. ¹H NMR was consistent with product. Anal. Calc'd. for C₂₅H₃₃N₆O₄F·2.75 HCl: C, 51.73; H, 6.07; N, 13.41. Found: C, 51.62; H, 5.74; N, 13.34.

### Example 2-25

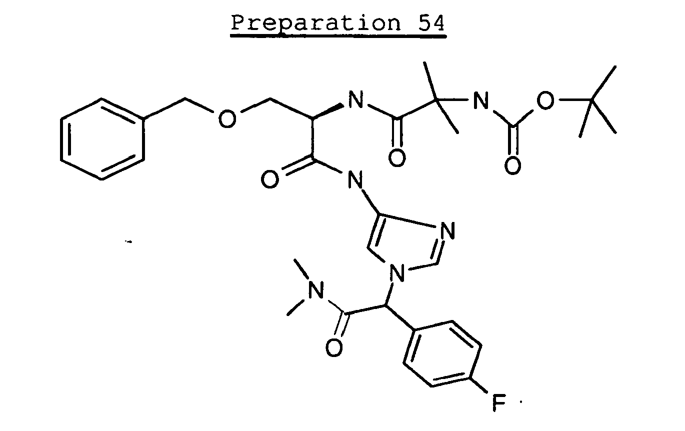

Reaction of the product of Preparation 10 (1.0 g, 1.7 mmol), dimethylamine hydrochloride (0.14 g, 1.7 mmol), triethylamine (0.26 mL, 1.9 mmol), 1-hydroxybenzotriazole (0.26 g, 1.9 mmol) and 1-(3-dimethylaminopropyl)-3-ethylcarbodiimide (0.4 g, 1.9 mmol) in dimethylformamide (30 mL) as described in Preparation EX4A gave 0.55 g (52%) of the desired product as a tan foam: ¹H-NMR is consistent with structure; MS (ion spray) 625.4 (M+1) ; Anal. Calc'd for C₃₂H₄₁FN₆O₆: C, 61.53; H, 6.61; N, 13.45. Found: C, 61.22; H, 6.33; N, 13.44.

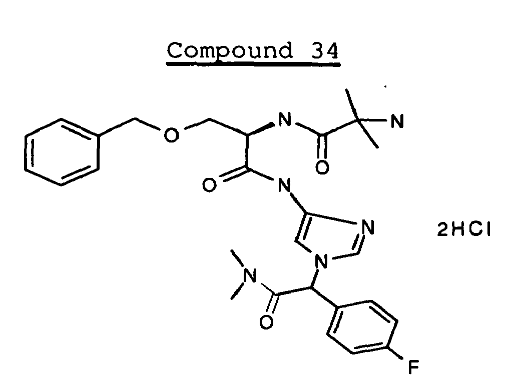

Reaction of the product of Preparation 54 (0.54 g, 0.86 mmol) and trifluoroacetic acid (2 mL), dichloromethane (6 mL) as described in Example 2-7 gave 0.4 g (77%) of the desired product as a mixture of isomers: ¹H-NMR is consistent with structure. MS (ion spray) 525.4 (M+1); Anal. Calc'd for C₂₇H₃₃FN₆O₆·2HCl: C, 54.27; H, 5.90; N, 14.06. Found: C, 53.11; H, 5.70; N, 13.58.

### Example 2-26

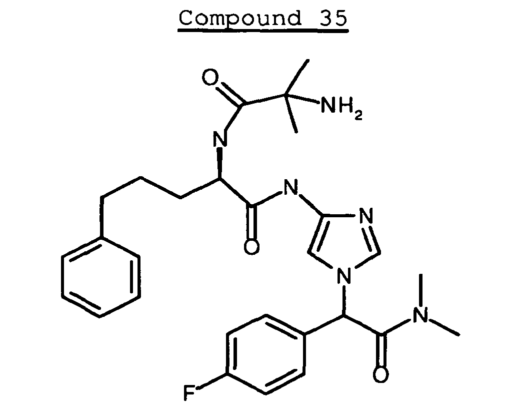

To a solution of the product of Preparation 75 (1.45 g, 2.29 mmol) stirring at room temperature in dichloromethane (50 mL) was added trifluoroacetic acid (15 mL). After 3 hours, the mixture was concentrated and the material treated with excess aqueous sodium bicarbonate. The aqueous mixture was extracted with ethyl acetate and the combined organic extracts concentrate. The resulting residue was purified by flash chromatography (silica gel, chloroform/methanol) to provide 1.55 g of the desired product: ESMS: (M+H)⁺ 523.3. The isomeric mixture (3.44 g) was separated as previously described in Example 2-9 to provide 0.98 g of pure isomer 1 (t_{R} = 7.94 min) and 0.81 g of isomer 2 (t_{R} = 10.57 min). For isomer 2, 0.80 g (1.53 mmol) was dissolved in ethyl acetate/methanol and treated with a saturated solution of hydrochloric acid in diethyl ether. The resulting mixture was concentrated to provide 0.90 g (92%) of the desired product as a light tan solid: ESMS: (M+H)⁺ 523.4, 524.5. ¹H NMR was consistent with product. *Anal.* Calc'd. for C₂₈H₃₅N₆O₃F·3.25 HCl: C, 52.46; H, 6.01; N, 13.11. Found: C, 52.49; H, 6.23; N, 11.80.

### Compounds of Formula I

Compounds of Formula I were synthesized by methods similar to the foregoing. These compounds include those wherein:
a) R1 is C₆H₅(CH₂)₃-, R3 is phenyl para substituted by W, W is F, R4 is methyl, and Y is pyrrolidin-1-yl,
b) R1 is C₆H₅CH₂OCH₂-, R3 is phenyl para substituted by W, W is F, R4 is methyl, and Y is pyrrolidin-1-yl,
c) R1 is C₆H₅(CH₂)₃-, R3 is phenyl para substituted by W, W is F, R4 is methyl, and Y is 4-methylpiperidin-1-yl,

### Example 3

### Pituitary Cell Culture Assay for Growth Hormone Secretion

Thirty-two 250 g male Sprague-Dawley rats were used for each assay. The animals were killed by decapitation and anterior pituitaries were removed and placed into ice cold culture medium. The pituitaries were sectioned into eighths and enzymatically digested using trypsin (Sigma Chemical) to weaken connective tissue. Pituitary cells were dispersed by mechanical agitation, collected, pooled and then seeded into 24-well plates (300,000 cells/well). After 4 days of culture, the cells formed an even monolayer. Cells were then washed with medium and challenged to secrete GH by the addition of GH secretagogues to the medium. After 15 min at 37 °C, the medium was removed and stored frozen until radioimmunoassays for rat GH were performed. Doses of secretagogue were added in quadruplicate. Representative data is provided in Table 1 below. Compounds disclosed herein are active in the assay as described. Both EC₅₀ and efficacy values were calculated by the 4-parameter logistic equation. Such values were pooled and represented as mean +/- standard error, when appropriate.

**Table 1**

| EXAMPLES | GH |
|---|---|
| PART 1 | secretion |
| Example # | EC₅₀ (µM) |
| 6 | 5.53 |
| 8 | 2.39 |

## Claims

1. A compound of the formula I 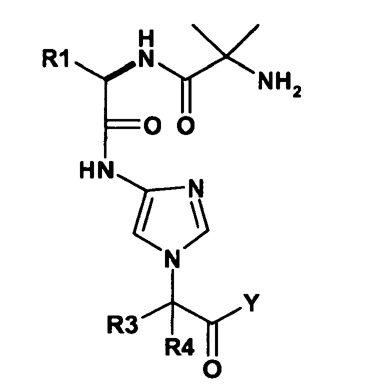 wherein:
R¹ is C₆H₅CH₂OCH₂-, C₆H₅(CH₂)₃- or indol-3-ylmethyl;
Y is pyrrolidin-1-yl, 4-methyl piperidinyl or NR2R2;
R2 are each independently a C₁ to C₆ alkyl;
R3 is phenyl para-substituted by W;
W is F; and
R4 is CH₃,
or a pharmaceutically acceptable salt or solvate thereof.

2. A compound of Claim 1 wherein said compound has the (R,R) stereo configuration.

3. A compound as claimed in Claim 2 wherein
R1 is C₆H₅(CH₂)₃-, R3 is phenyl para substituted by W, W is F, R4 is methyl, and Y is pyrrolidin-1-yl, or
R1 is C₆H₅CH₂OCH₂-, R3 is phenyl para substituted by W, W is F, R4 is methyl, and Y is pyrrolidin-1-yl, or
R1 is C₆H₅(CH₂)₃-, R3 is phenyl para substituted by W, W is F, R4 is methyl, and Y is 4-methylpiperidin-1-yl.

4. A compound according to claim 1 having the formula 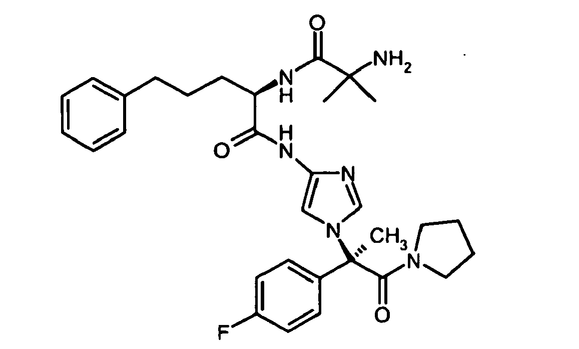 or a pharmaceutically acceptable salt or solvate thereof.

5. A compound according to claim 1 having the formula 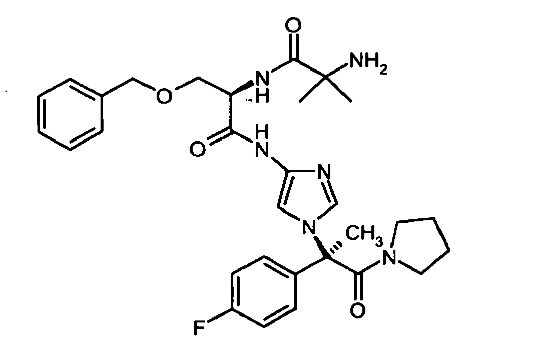 or a pharmaceutically acceptable salt or solvate thereof.

6. A compound according to claim 1 having the formula 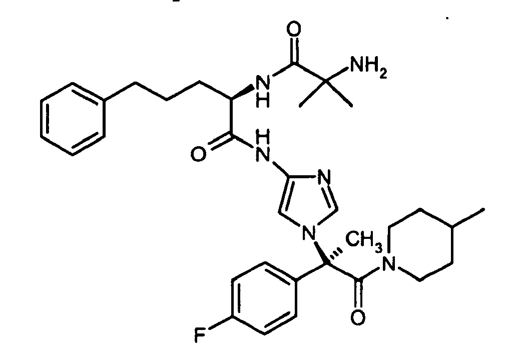 or a pharmaceutically acceptable salt or solvate thereof.

7. A pharmaceutical formulation which comprises, as an active ingredient a compound according to any one of claims 1 to 6, or a pharmaceutically acceptable salt or solvate thereof, associated with one or more pharmaceutically acceptable carriers, diluents, or excipients.

8. A pharmaceutical formulation according to Claim 7 which further comprises a bone-antiresorptive agent.

9. A formulation according to Claim 8 wherein said bone antiresorptive agent is a bisphosphonate.

10. A compound as claimed in any one of claims 1 to 6, for use in therapy.

11. A compound as claimed in any one of claims 1 to 6, for use in increasing the level of endogenous growth hormone in a human or an animal.

12. A compound as claimed in any one of claims 1 to 6, for use in the treatment or prevention of a physiological condition which may be modulated by an increase in endogenous growth hormone.

13. The use of a compound as claimed in any one of claims 1 to 6, for the manufacture of a medicament for the treatment or prevention of congestive heart failure, osteoporosis and/or loss of muscle strength.

## Patentansprüche

1. Verbindung der Formel I 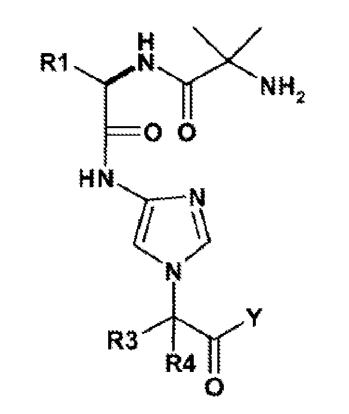 worin
R1 für C₆H₅CH₂OCH₂-, C₆H₅(CH₂)₃- oder Indol-3-ylmethyl steht,
Y für Pyrrolidin-1-yl, 4-Methylpiperidinyl oder NR2R2 steht,
R2 jeweils unabhängig für C₁-C₆-Alkyl steht,
R3 für Phenyl steht, in para-Stellung durch W substituiert ist,
W für F steht, und
R4 für CH₃ steht,
oder ein pharmazeutisch akzeptables Salz oder Solvat hiervon.

2. Verbindung nach Anspruch 1, worin diese Verbindung die Stereokonfiguration (R,R) hat.

3. Verbindung nach Anspruch 2, worin
R1 für C₆H₅(CH₂)₃- steht, R3 für Phenyl steht, das in para-Stellung durch W substituiert ist, wobei W für F steht, R4 für Methyl steht und Y für Pyrrolidin-1-yl steht, oder
R1 für C₆H₅-CH₂OCH₂- steht, R3 für Phenyl steht, das in para-Stellung durch W substituiert ist, wobei W für F steht, R4 für Methyl steht und Y für Pyrrolidin-1-yl steht, oder
R1 für C₆H₅(CH₂)₃- steht, R3 für Phenyl steht, das in para-Stellung durch W substituiert ist, wobei W für F steht, R4 für Methyl steht und Y für 4-Methylpiperidin-1-yl steht.

4. Verbindung nach Anspruch 1 der Formel 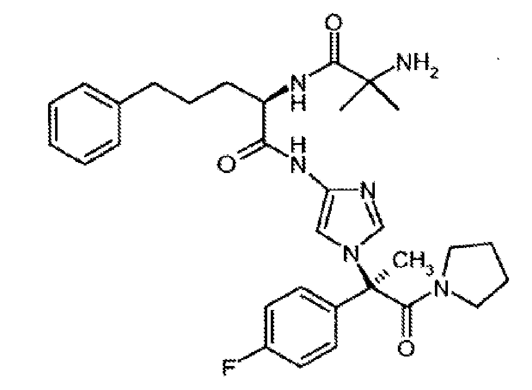 oder ein pharmazeutisch akzeptables Salz oder Solvat hiervon.

5. Verbindung nach Anspruch 1 der Formel 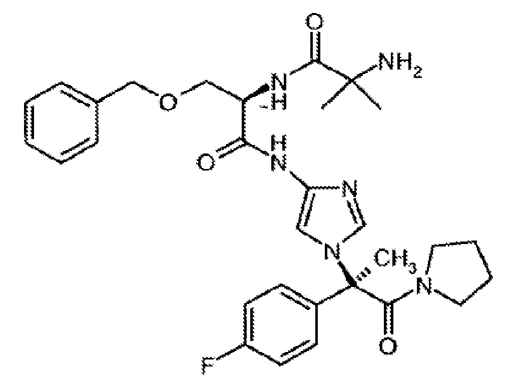 oder ein pharmazeutisch akzeptables Salz oder Solvat hiervon.

6. Verbindung nach Anspruch 1 der Formel 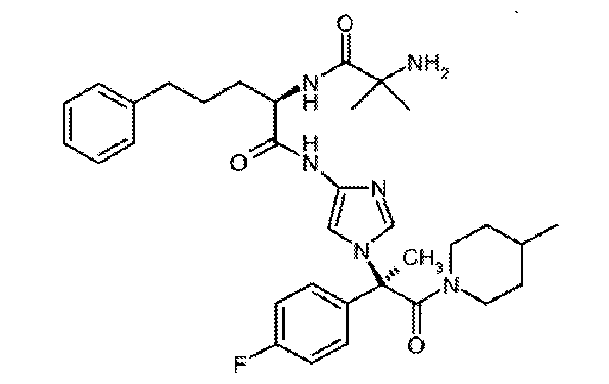 oder ein pharmazeutisch akzeptables Salz oder Solvat hiervon.

7. Pharmazeutische Formulierung, umfassend als einen Wirkstoff eine Verbindung nach einem der Ansprüche 1 bis 6 oder ein pharmazeutisch akzeptables Salz oder Solvat hiervon in Assoziation mit ein oder mehr pharmazeutisch akzeptablen Trägern, Verdünnungsmitteln oder Exzipientien.

8. Pharmazeutische Formulierung nach Anspruch 7, die ferner ein antiresorptives Mittel für Knochen umfasst.

9. Formulierung nach Anspruch 8, worin das antiresorptive Mittel für Knochen ein Bisphosphonat ist.

10. Verbindung nach einem der Ansprüche 1 bis 6 zur Verwendung in der Therapie.

11. Verbindung nach einem der Ansprüche 1 bis 6 zur Verwendung für eine Erhöhung des Spiegels an endogenem Wachstumshormon bei einem Menschen oder einem Tier.

12. Verbindung nach einem der Ansprüche 1 bis 6 zur Verwendung für die Behandlung oder Prävention eines physiologischen Zustands, der durch eine Erhöhung an endogenem Wachstumshormon moduliert sein kann.

13. Verwendung einer Verbindung nach einem der Ansprüche 1 bis 6 zur Herstellung eines Arzneimittels für die Behandlung oder Prävention von kongestivem Herzversagen, Osteoporose und/oder Verlust an Muskelstärke.

## Revendications

1. Composé de formule 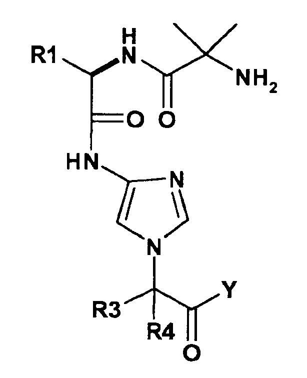 dans laquelle :
R¹ représente C₆H₅CH₂OCH₂-, C₆H₅(CH₂)₃- ou un groupe indol-3-ylméthyle;
Y représente un groupe pyrrolidin-1-yle, 4-méthyl-pipéridinyle ou NR₂R_{2;}
R² représente chacun indépendamment un groupe alkyle en C₁ à C₆;
R³ représente un groupe phényle substitué en para par W;
W représente F; et
R⁴ représente un groupe CH₃,
ou un sel ou solvate de celui-ci acceptable sur le plan pharmaceutique.

2. Composé selon la revendication 1, dans lequel ledit composé possède la configuration stéréo (R, R).

3. Composé selon la revendication 2, dans lequel
R¹ représente C₆H₅(CH₂)₃-, R³ représente un groupe phényle substitué en para par W, W représente F, R⁴ représente un groupe méthyle, et Y représente un groupe pyrrolidin-1yle, ou bien
R¹ représente C₆H₅CH₂OCH₂-, R³ représente un groupe phényle substitué en para par W, W représente F, R⁴ représente un groupe méthyle, et Y représente un groupe pyrrolidin-1yle, ou bien
R¹ représente C₆H₅(CH₂)₃, R³ représente un groupe phényle substitué en para par W, W représente F, R⁴ représente un groupe méthyle, et Y représente un groupe 4-méthylpipéridin-1-yle.

4. Composé selon la revendication 1, possédant la formule 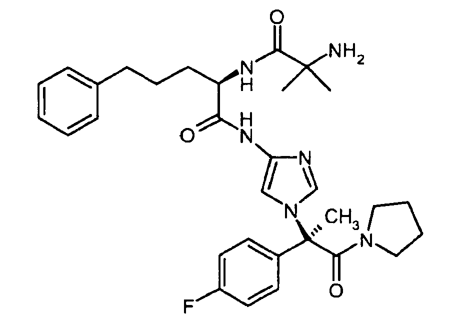 ou un sel ou solvate de celui-ci, acceptable sur le plan pharmaceutique.

5. Composé selon la revendication 1, possédant la formule 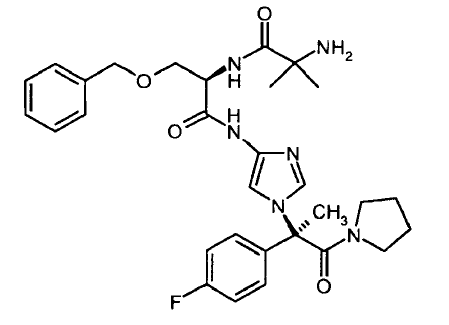 ou un sel ou solvate de celui-ci, acceptable sur le plan pharmaceutique.

6. Composé selon la revendication 1, possédant la formule 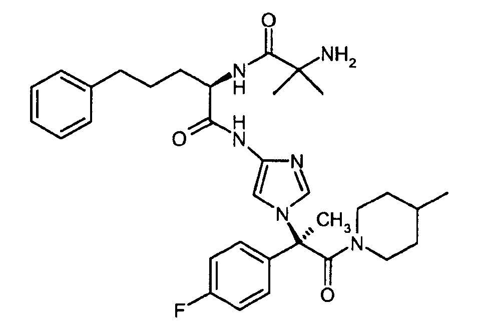 ou un sel ou solvate de celui-ci, acceptable sur le plan pharmaceutique.

7. Formulation pharmaceutique qui comprend, en tant qu'ingrédient actif, un composé selon l'une quelconque des revendications 1 à 6, ou un sel
ou solvate de celui-ci, acceptable sur le plan pharmaceutique, associé à un ou plusieurs véhicules, diluants, ou excipients, acceptables sur le plan pharmaceutique.

8. Formulation pharmaceutique selon la revendication 7, qui comprend en outre un agent inhibiteur de résorption osseuse.

9. Formulation selon la revendication 8, dans laquelle ledit agent inhibiteur de résorption osseuse est un biphosphonate.

10. Composé selon l'une quelconque des revendications 1 à 6, destiné à une utilisation en thérapie.

11. Composé selon l'une quelconque des revendications 1 à 6, destiné à être employé pour augmenter la teneur en hormone de croissance endogène chez un humain ou un animal.

12. Composé selon l'une quelconque des revendications 1 à 6, destiné à être employé dans le traitement ou la prévention d'une condition physiologique qui peut être modulée par une augmentation d'hormone de croissance endogène.

13. Utilisation d'un composé selon l'une quelconque des revendications 1 à 6, destiné à la fabrication d'un médicament pour le traitement ou la prévention de l'insuffisance cardiaque congestive, de l'ostéoporose et/ou de la perte de force musculaire.
